# EUROPEAN PATENT SPECIFICATION

(11) **EP 0 533 701 B1**
(45) Date of publication and mention of the grant of the patent: **24.08.1994**
(21) Application number: 91910177.4
(22) Date of filing: 14.05.1991
(51) Int. Cl.: C12N 15/75, C12N 15/32

(54) **SHUTTLE VECTOR FOR RECOMBINANT $i(BACILLUS THURINGIENSIS) STRAIN DEVELOPMENT**
PENDELVEKTOR FÜR DIE ENTWICKLUNG VON EINEM REKOMBINANTEN BACILLUS THURINGIENSIS-STAMM
VECTEUR NAVETTE SERVANT A LA CULTURE DE SOUCHES DE $i(BACILLUS THURINGIENSIS) DE RECOMBINAISON

(30) Priority: 15.05.1990 US 523671
(43) Date of publication of application: 31.03.1993
(73) Proprietor: ECOGEN INC, Langhorne, PA 19047-1810 (US)
(72) Inventor: BAUM, James, A., Doylestown, PA 18901 (US)
(74) Representative: VOSSIUS & PARTNER
(86) International application number: US9103360
(87) International publication number: WO9118102

(56) References cited:
- EP-A- 342 633
- FEMS MICROBIOLY LETTERS, vol. 60, 15 July 1989, ELSEVIER PUBLISHERS,pp. 211-218; LERECLUS, D. et al.
- APPLIED AND ENVIRONMENTAL MICROBIOLOGY, vol. 56, no. 11, November 1990, AMERICAN SOCIETY FOR MICROBIOLOGY, pp. 3420 -3428, BAUM J.A. et al.

## Description

### Field of the Invention

The present invention relates to a bifunctional recombinant DNA cloning vector for use in *Bacillus thuringiensis* and *E.coli* strains. The vectors are useful in the development of recombinant *Bacillus thuringiensis* strains that contain one or more insecticidal toxin genes introduced from other *Bt* strains.

### Background of the Invention

*Bacillus thuringiensis* ("*Bt*") is a gram-positive soil bacterium that produces proteinaceous crystalline inclusions during sporulation. These *Bt* crystal proteins are often highly toxic to specific insects. Insecticidal activities have been identified for crystal proteins from various *Bt* strains against insect larvae from the insect orders Lepidoptera (caterpillars), Diptera (mosquitos, flies) and Coleoptera (beetles).

Individual *Bt* crystal proteins, also called delta-endotoxins or parasporal crystals or toxin proteins, can differ extensively in their structure and insecticidal activity. These insecticidal proteins are encoded by genes typically located on large plasmids, greater than 30 megadaltons (mDa) in size, that are found in *Bt* strains. A number of these *Bt* toxin genes have been cloned and the insecticidal crystal protein products characterized for their specific insecticidal properties. A good review of cloned *Bt* toxin genes and crystal proteins is given by Höfte et al.*, Microbiol.Rev.* (1989) 53:242-255 (hereinafter Höfte and Whiteley, 1989), who also propose a useful nomenclature and classification scheme that has been adopted in this disclosure.

The insecticidal properties of *Bt* have been long recognized, and *Bt* strains were first commercially introduced in biological insecticide products in the 1960's. These insecticide formulations typically contain dried *Bt* fermentation cultures whose crystal protein is toxic to lepidopteran insect species. Most of the current commercialized *Bt* insecticides are derived from "wild-type" *Bt* strains, i.e., purified cultures of native *Bt* strains isolated from natural sources.

Several newly commercialized *Bt* strains are genetically altered strains that have increased insecticidal potency as well as insecticidal activity against a broader spectrum of target insects, as compared with the parent *Bt* strains. Such strains are exemplified in International Patent Publication No. WO 88/08877, published November 17, 1988 by applicant Ecogen Inc.

Development of these genetically altered *Bt* strains does not involve recombinant DNA technology but is instead based on the techniques of plasmid conjugal transfer, which is a natural form of genetic exchange between bacteria, and of plasmid curing, in which certain nonessential plasmids are deleted from a bacterium.

Plasmid conjugal transfer, or conjugation, is limited by the fact that many plasmids carrying useful toxin genes are not amenable to transfer from their native host *Bt* strain to another "recipient" *Bt* strain. Furthermore, some plasmids which can be transferred by conjugation are inherently incompatible with other plasmids, so a stable "transconjugant" *Bt* strain, containing the two desired, incompatible plasmids, cannot be realized.

Another drawback to conjugation is that some mobilizable, or transferable, plasmids carry undesirable toxin genes in addition to the desired gene, so the quantity of the desired crystal protein produced is limited by concurrent production of an unwanted crystal protein.

Despite the demonstrated efficacy of commercialized transconjugant *Bt* strains against certain target insects, there is a clear need for improved *Bt* strains against other insect pests. Development of such *Bt* strains will be facilitated by use of recombinant DNA technology in *Bt* strain construction.

Recombinant DNA procedures provide great flexibility in the construction of novel plasmids containing one or more toxin genes, by permitting selection, manipulation and control of crystal protein type and production and of gene regulation and expression. Some techniques for utilizing the recombinant DNA approach in the production of transformed *Bt* strains are described in European Patent Application No. EP 0 342 633-A2, published November 23, 1989 by applicant Ciba-Geigy AG.

The recombinant *Bt* strains disclosed in EP 0 342 633-A2 and other publications are generally characterized by the presence of one or more antibiotic resistance marker genes on the recombinant plasmid harboring the desired *Bt* toxin gene(s). Such antibiotic resistance marker genes provide a means for the identification and selection of transformed *Bt* strains containing the recombinant toxin-encoding plasmid but are undesirable in viable *Bt* strains developed for use in commercial insecticide formulations. Since antibiotic resistance genes are not ordinarily present in native *Bt* strains, pesticide and environmental regulatory agencies may be reluctant to approve antibiotic-resistant recombinant *Bt* strains for unrestricted environmental release and for use in biological insecticide formulations.

A major reason for the presence of antibiotic resistance genes in recombinant *Bt* strains described in the literature is the use of bifunctional cloning vectors containing such resistance marker genes. Portions of these cloning vectors are typically derived from plasmids not native to *Bt*, e.g., *Escherichia coli, Bacillus cereus, B. subtilis* or *Staphylococcus aureus* plasmids, and contain, in addition to the antibiotic resistance marker gene, an origin of replication from a non-*Bt* source that is also functional in *Bt* and therefore permits the cloning vector to be replicated in *Bt*.

Miller et al., U.S. Patent No. 4,503,155 granted on March 5, 1985 disclose such multifunctional cloning vectors useful in *Streptomyces, Bacillus* and *E.coli.*

Several recent literature references report *Bt* cloning vectors that employ origins of replication from *Bt* plasmids, but these also contain antibiotic resistance marker genes and other non-*Bt* DNA elements that are not intended to be readily removable.

Miteva et al., *Arch.Microbiol.* (1988) *150*:496-498, report the cloning in *E.coli* and mapping of a small cryptic plasmid from *Bacillus thuringiensis* var. *israelensis.* The resultant bifunctional vector, although able to replicate in both *E.coli* and *Bt*, exhibited poor segregational stability in *Bt israelensis*.

Mahillon et al., *Plasmid* (1988) *19*:169-173, describe the cloning in *E.coli* and restriction mapping of three small cryptic plasmids from *Bacillus thuringiensis* var. *thuringiensis.* One of these plasmids was pGI2. The authors speculate that the cloned plasmids could serve as the starting point for the development of new shuttle vectors for *Bacillus thuringiensis.* Mahillon et al., *Nucl.Acids Res*. (1988) *16*:11827-11828, report the complete nucleotide sequence of pGI2, a small cryptic plasmid cloned from *Bacillus thuringiensis* var.*thuringiensis.* pGI2 was shown to contain the transposon TN4430, which represented over 40% of the molecule.

Lereclus et al., *FEMS Microbiol.Lett.* (1988) *49*:417-422, describe the characterization of two small cryptic *Bt* plasmids cloned in *E.coli*. The replication origins for these two plasmids were localized to DNA fragments of 2.1 and 2.8 kilobases (kb). One of the small cryptic plasmids, pHT1030, exhibited good segregational stability in *Bacillus subtilis*; plasmids derived from pHT1030 were stably maintained, with 90% recovery after 30 generations of growth at 30°C in the absence of selection with antibiotic.

In a follow-up article, Lereclus et al., *FEMS Microbiol.Lett.* (1989) *60*:211-217, describe the construction of shuttle vector derived from the small *Bt* resident plasmid pHT1030. This shuttle vector, designated pHT3101, was used to determine suitable conditions for efficient transformation of *Bt* by electroporation. A *Bt* toxin gene, which was expressed poorly on its native *Bt* plasmid, was cloned using this shuttle vector and shown to express both in *E.coli* and in *Bt*.

Schurter et al., *Mol.Gen.Genet.* (1989) *218*:177-181 and in European Patent Application No. 0 342 633-A2 published November 23, 1989, describe the use of electroporation to efficiently transform acrystalliferous *B.thuringiensis* and *B.cereus* strains with a shuttle vector containing a *Bt* endotoxin gene. The studies utilized a bifunctional shuttle vector capable of replicating in both *E.coli* and *Bacillus* spp. and constructed from a variety of bacterial plasmid sources, none of which appear to be *Bt*.

The novel shuttle vector of this invention facilitates the incorporation of recombinant plasmids into Bt strain constructs that contain no DNA derived from *E.coli* or other non-*Bt* biological sources. Using the shuttle vector of this invention, transformed *Bt* strains may be constructed that are free of antibiotic resistance marker genes as well as free of non-*Bt* replication origins, e.g., *E.coli*-functional replication origins.

The shuttle vector of this invention thus provides the basis for development of improved biological pesticides, containing viable recombinant *Bt* strains which have insecticidal properties superior to presently-available commercial, genetically altered *Bt* strains. These improved, recombinant *Bt* strains are expected to be suitable for unrestricted environmental release, since they are essentially free of foreign DNA from non-*Bt* biological sources and contain toxin genes and other plasmid DNA derived solely from *Bt* source strains.

### Summary of the Invention

The plasmid shuttle vector of this invention, useful for introducing genes into a *Bacillus thuringiensis* host, is a plasmid capable of transforming a *Bt* bacterium and contains (i) a removable DNA restriction fragment containing an origin of replication functional i*n E. coli*; (ii) a multiple cloning site, with multiple restriction endonuclease cleavage sites to facilitate cloning of an inserted *Bt* gene, and (iii) a removable origin of replication derived from a *Bt* plasmid, the *Bt*-derived origin of replication being adjacent to the multiple cloning site and these latter two elements having flanking restriction endonuclease cleavage sites that enable these elements to be isolated from the plasmid shuttle vector as a single DNA fragment and to be introduced, after self-ligation, as a functional plasmid into a *Bt* host.

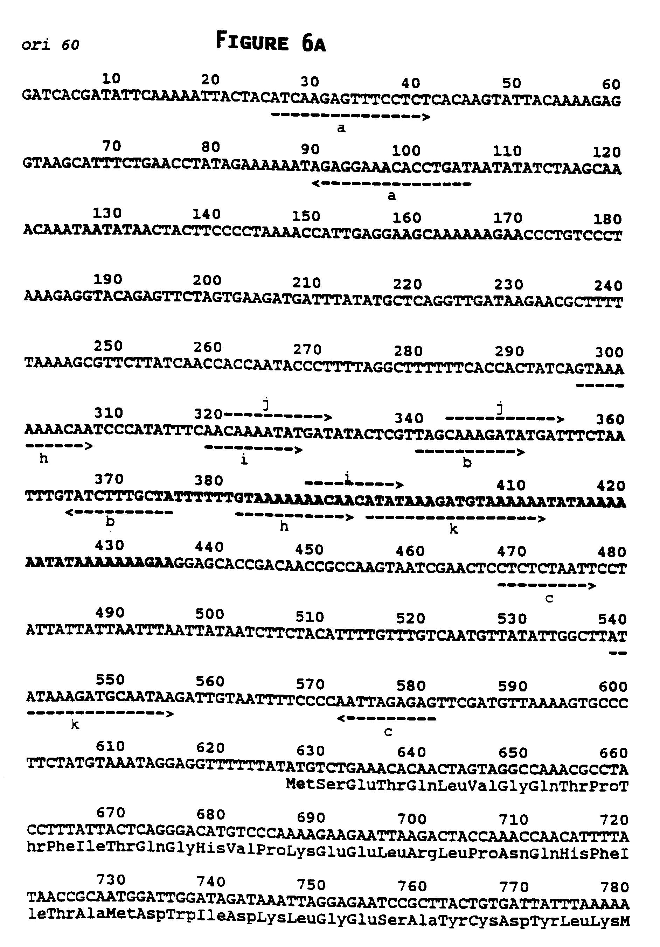

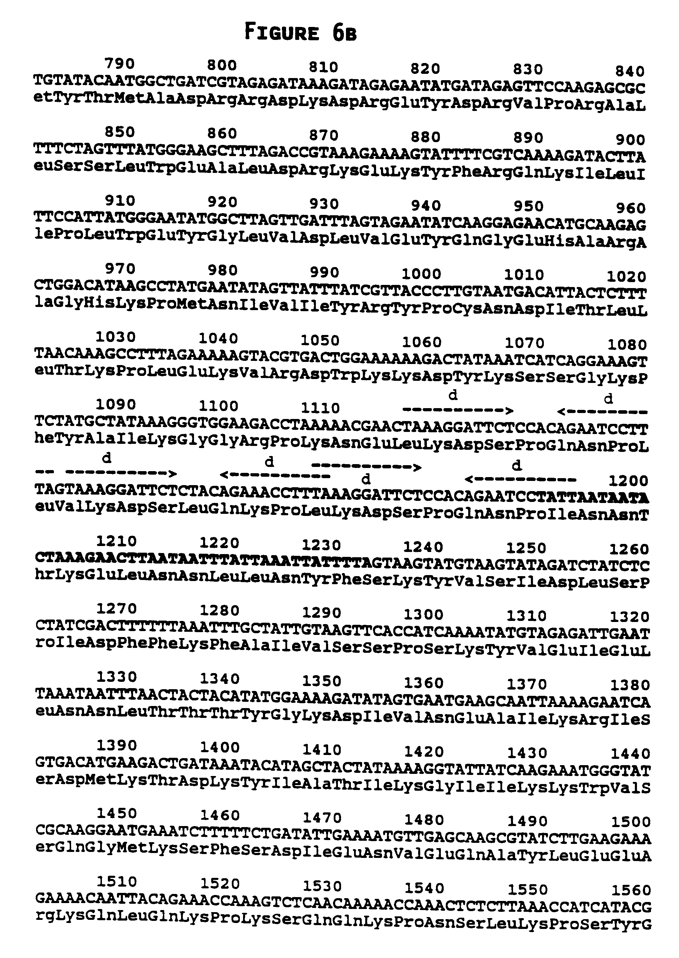

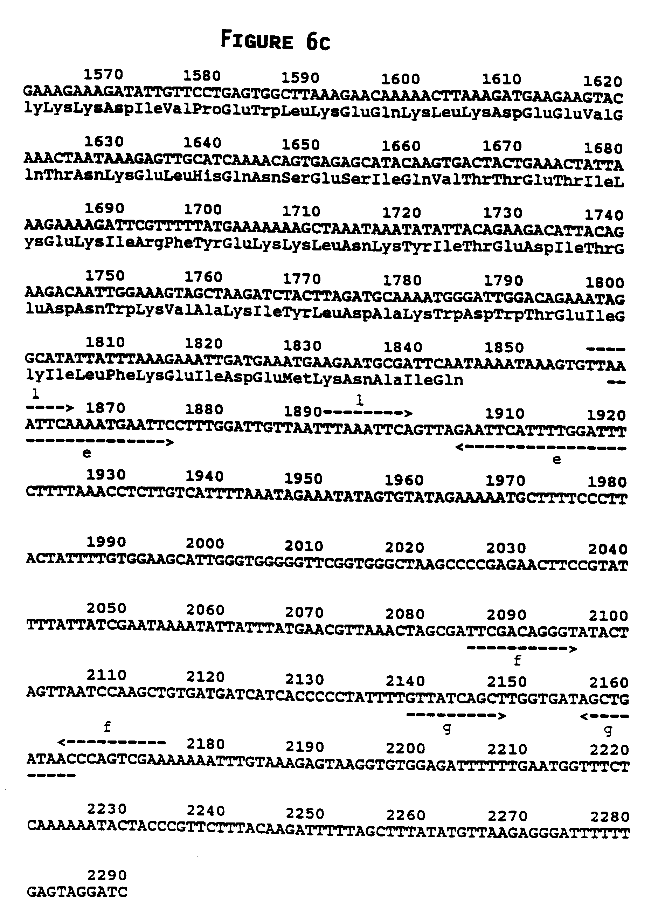

The plasmid shuttle vector of this invention contains a *Bt*-derived origin of replication selected from the group of *ori 44*, *ori 60* and *ori 43*, whose respective nucleotide sequences are shown in Figures 5, 6, and 7. Other *Bt*-derived replication origins may also be used in place of *ori 44*, *ori 60*, or *ori 43*.

The flanking restriction endonuclease cleavage sites in the plasmid shuttle vector may be identical, such sites being unique to the plasmid shuttle vector and being absent from a gene of interest to be introduced into a *Bt* host via the shuttle vector. Preferred flanking restriction endonuclease cleavage sites are *SalI* and *SfiI* sites. The plasmid shuttle vector may also contain two sets of flanking restriction endonuclease cleavage sites, one pair of flanking restriction sites being *Sal*I sites and the other pair of flanking restriction sites being *Sfi*I sites.

The plasmid shuttle vector may also contain two identical restriction endonuclease cleavage sites that flank the removable DNA restriction fragment containing the origin of replication functional in *E.coli*, such sites being unique to the plasmid shuttle vector and being absent from a gene of interest to be introduced into a *Bt* host via the shuttle vector. These two identical restriction endonuclease cleavage sites flanking the *E.coli*-functional origin of replication are preferably *Not*I sites. These flanking restriction sites facilitate removal of the DNA restriction fragment containing the *E.coli*-functional origin of replication.

In addition to all of these restriction sites, the plasmid shuttle vector may also contain two identical restriction endonuclease cleavage sites that flank the removable *Bt*-derived origin of replication, such sites being unique to the plasmid shuttle vector. These two identical restriction endonuclease cleavage sites flanking the removable *Bt*-derived origin of replication are preferably *Xba*I sites. These flanking restriction sites facilitate removal of the *Bt*-derived origin of replication and insertion of a different *Bt*-derived origin of replication.

The shuttle vector may also contain at least one selectable marker gene conferring antibiotic resistance, the gene being present on a removable DNA restriction fragment in the plasmid. Selectable marker genes may be functional in *Bt* or functional in *E.coli* or in both. Separate or different marker genes may be desirable for separately selecting for transformants of *E.coli* and of *Bt*. If functional in *Bt*, the selectable marker gene is preferably located adjacent to the *Bt*-derived origin of replication. If functional in *E.coli*, the selectable marker gene is preferably located adjacent to or within the DNA fragment containing the origin of replication functional in *E.coli.* The selectable marker gene functional in *Bt* or *E.coli* may be one conferring resistance to chloramphenicol, tetracycline, kanamycin, or other appropriate antibiotics. Another selectable marker gene functional in *E.coli* is the ampicillin (beta lactamase) resistance gene.

The multiple cloning site in the plasmid shuttle vector of this invention is normally synthesized as a linked series of restriction endonuclease cleavage sites that facilitate cloning of an inserted *Bt* gene, i.e., a *Bt* gene that is intended to be functional as a toxin-encoding gene in a recombinant *Bt* strain construct.

A preferred multiple cloning site has restriction endonuclease cleavage sites in the following sequence, with the *Xba*I site being located adjacent to the *Bt*-derived origin of replication:
*Xba*I *Pst*I *Kpn*I *Sma*I *Avr*II *Bam*HI *Xho*I *Sst*I *Cla*I *Hpa*I *Sph*I *Eag*I *Sfi*I *Sal*I *Not*I.

Another preferred multiple cloning site has restriction endonuclease cleavage sites in the following sequence, with the *Bam*HI site being located adjacent to the *Bt*-derived origin of replication:
*Bam*HI *Xho*I *Pst*I *Sst*I *Sph*I *Sma*I *Hind*III *EcoR*I *Sal*I *Not*I.

These two multiple cloning sites are preferably employed with *ori 44*, *ori 60* or *ori 43*.

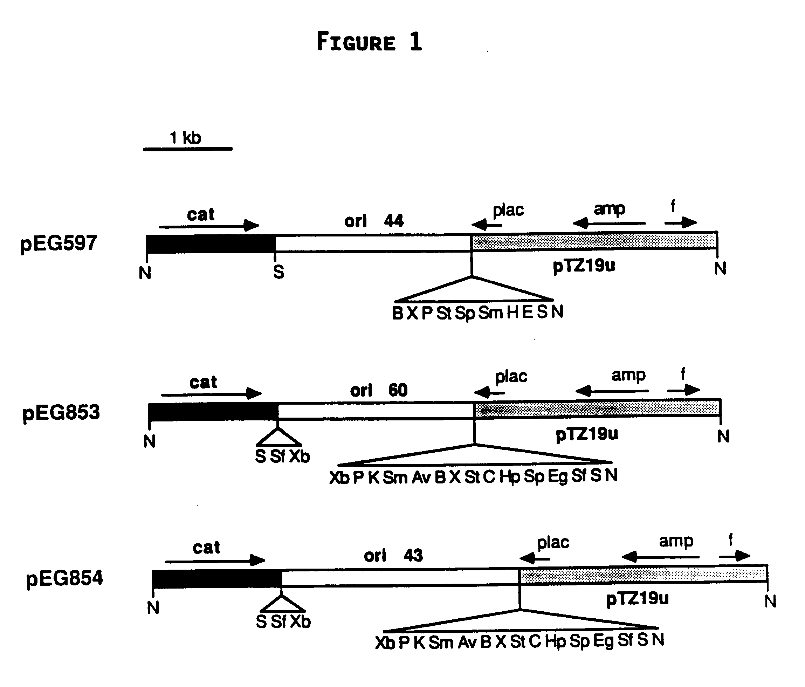

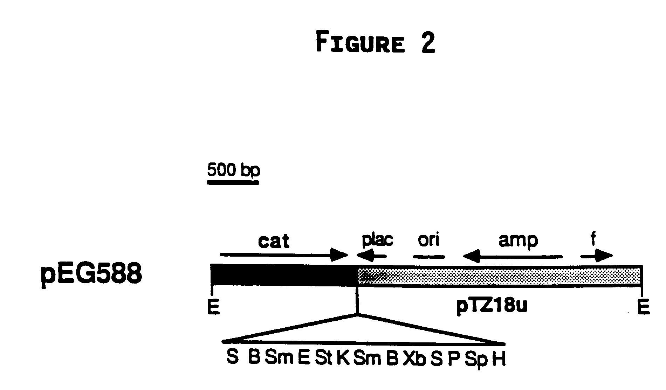

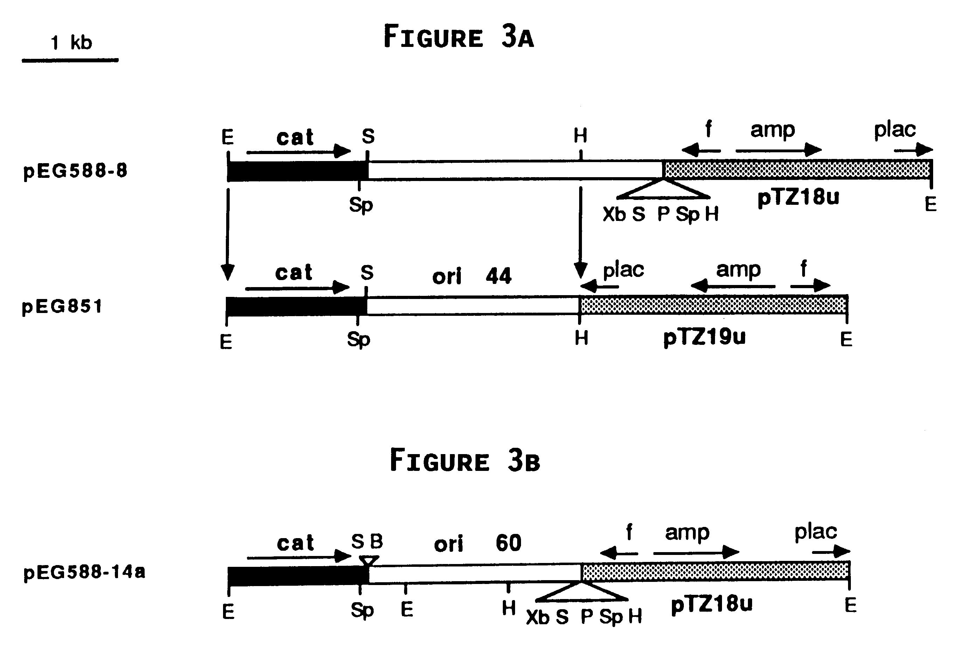

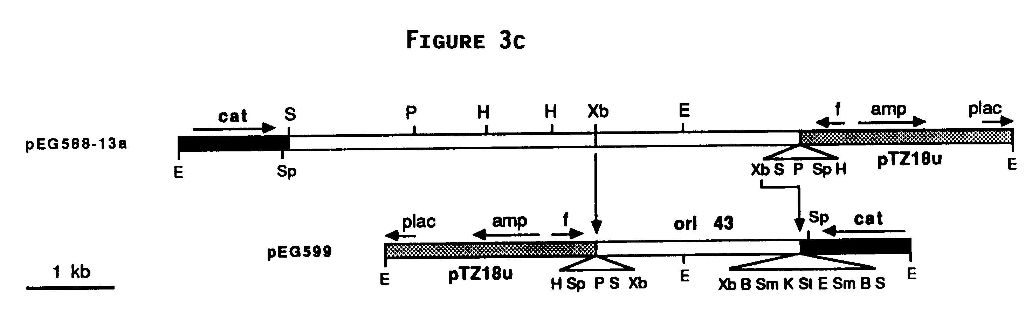

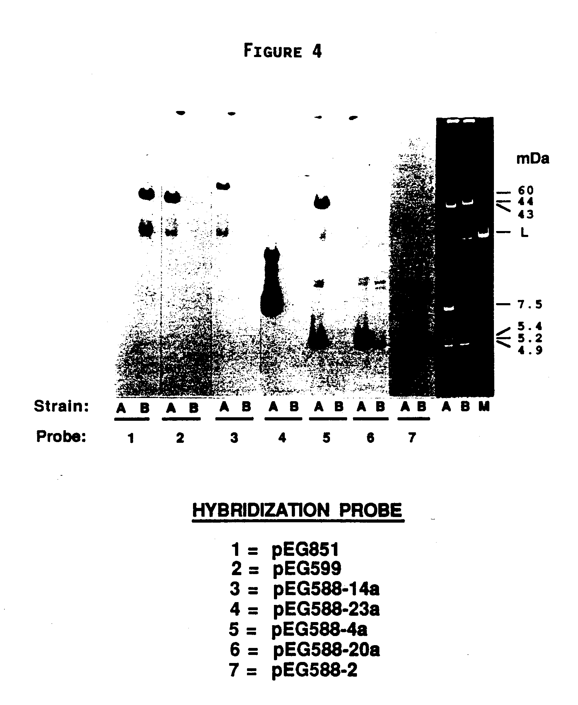

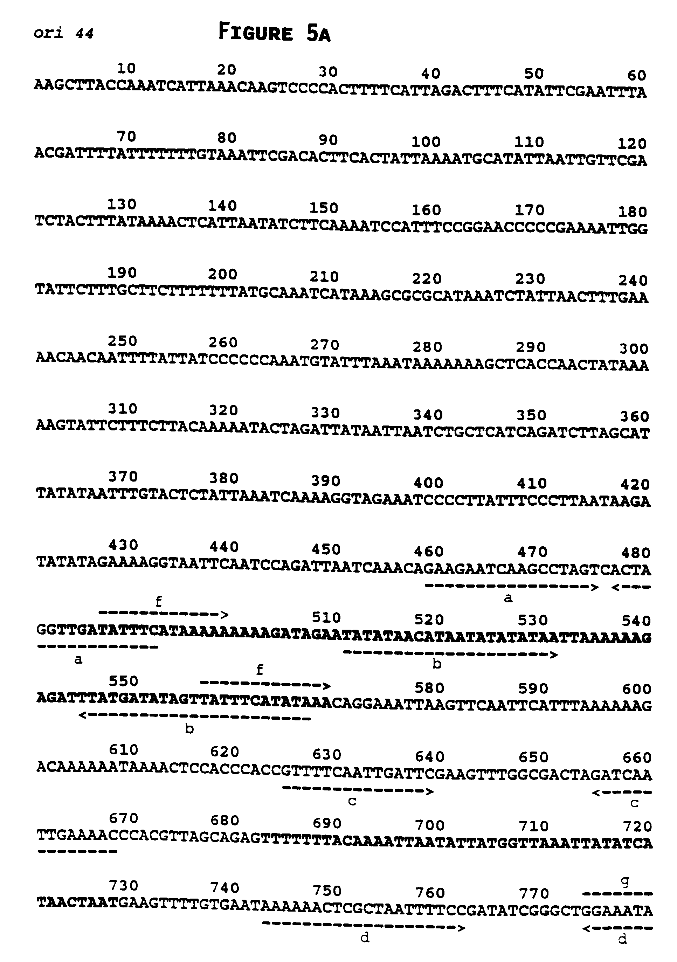

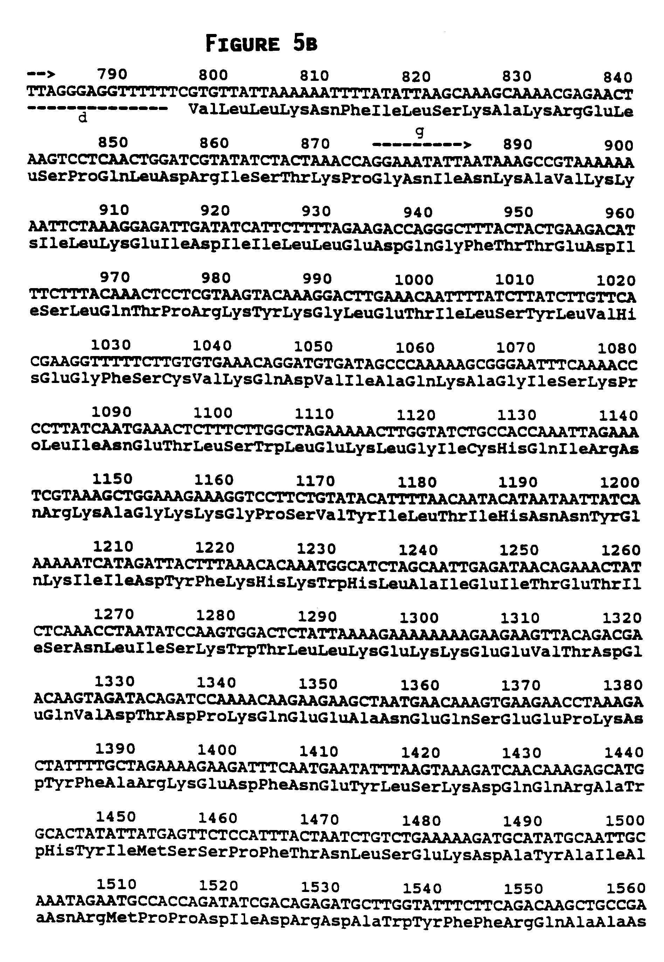

Three preferred plasmid shuttle vectors of this invention are shown in Fig. 1, which depicts the structural maps for vectors pEG597, pEG853 and pEG854. These shuttle vectors and their use are described in more detail in the Description of the Preferred Embodiments and in the Examples.

### Brief Description of the Drawings

Figure 1 depicts linear restriction maps for three plasmid shuttle vectors, pEG597, pEG853, and pEG854, and also illustrates their structural components. The individual vectors are shown opened at a *Not*I site located between the antibiotic resistance marker gene for chloramphenicol acetyltransferase (cat; solid segment) and a DNA fragment, pTZ19u (shaded segment), harboring an origin of replication (not shown) functional in *E.coli*, an f1 phage replication origin (f), an ampicillin antibiotic resistance marker gene (amp), and a *lac*Z promoter (plac). The *Bt*-derived origins of replication for each plasmid, *ori 44*, *ori 60* or *ori 43* (as indicated) are located on the DNA fragment shown as a white segment. The multiple cloning site (triangular segment) is located between the respective *Bt* replication origins and the pTZ19u DNA fragment. Letter abbreviations for the restriction endonuclease cleavage sites indicated in these structural maps may be deciphered as follows: Av=*Avr*II, B=*Bam*HI, C=*Cla*I, E=*Eco*RI, Eg=*Eag*I, H=*Hind*III, Hp=*Hpa*I K=*Kpn*I, N=*Not*I, P=*Pst*I, S=*Sal*I, Sf=*Sfi*I, Sm=*Sma*I, Sp=*Sph*I, St=*Sst*I, X=*Xho*I, Xb=*Xba*I. The "1 kb" symbol is a 1000 base pair length standard.

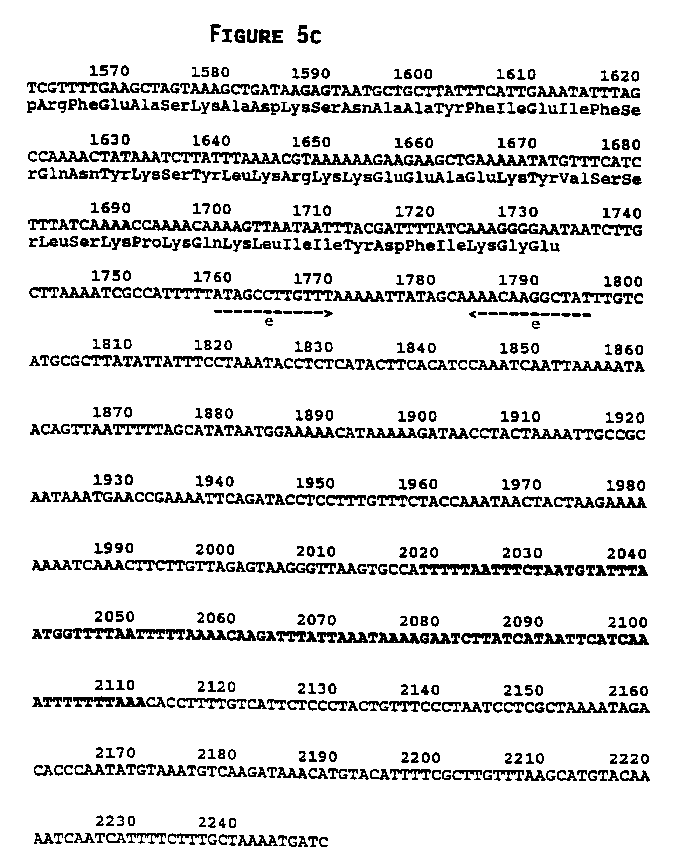

Figure 2 is a linear structural map of the replicon cloning vector pEG588, useful for recovering replication origins from *Bt* strains. The vector is shown opened at an *Eco*RI site (E) located between the antibiotic resistance marker gene for chloramphenicol acetyltransferase (cat; solid segment) derived from pMI1101 and a DNA fragment, pTZ18u (shaded segment), containing an origin of replication (ori) functional in *E.coli*, an f1 phage replication origin (f), an ampicillin antibiotic resistance marker gene (amp) and a *lac*Z promoter (plac). The multiple cloning site is located between the cat resistance gene and the pTZ18u DNA fragment and has restriction endonuclease cleavage sites whose letter abbreviations are deciphered in the summary for Figure 1. The "500 bp" symbol is a 500 base pair length standard.

Figure 3 shows linear structural maps for several recombinant plasmids containing *Bt* origin of replication *ori* 44, *ori* 60 or *ori 43*. In Figure 3A, pEG588-8 and its subclone, pEG851, carry the *Bt*-derived origin of replication *ori 44*. In Figure 3B, pEG588-14a carries *ori 60*. In Figure 3C, pEG588-13a and its subclone, pEG599, carry *ori 43*. The *Bt*-derived origins of replication are located on the DNA fragment shown as a white segment in the respective plasmids. The solid segment in each plasmid indicates the chloramphenicol acetyltransferase gene (cat), derived from pMI1101. The shaded segment in each plasmid is DNA fragment pTZ18u or pTZ19u (as indicated) containing an origin of replication functional in *E.coli,* a f1 phage replication origin (f), an ampicillin resistance gene (amp) and a *lac*Z promoter (plac). Letter abbreviations for the restriction endonuclease cleavage sites in these structural maps are deciphered in the summary for Figure 1. The "1 kb" symbol is a 1000 base pair length standard.

Figure 4 is a photograph of an autoradiogram and an ethidium bromide stained gel from a Southern blot analysis of resident plasmids in *Bt* var. *kurstaki* strains HD263-6 (Strain A) and HD73-26-10 (Strain B). The *Bt* replication origin clones listed in Table 1, representing seven different *Bt* plasmid replication origins, were used as hybridization probes; these are identified for each of the gel lanes. Abbreviations: L = linear DNA fragments; M = linear lambda DNA used as a size standard; mDa = megadaltons.

Figure 5 consists of Figures 5A, 5B and 5C and depicts the nucleotide sequence for *ori 44*, the *Bt* origin of replication derived from the 44 mDa plasmid of *Bt* var. *kurstaki* strain HD-263. The deduced amino acid sequence for the open reading frame extending from nucleotide position 797 to 1732 is also shown. DNA sequences rich in AT content (>85% AT) are indicated in boldface type. Direct and inverted repeat sequences are indicated by arrows and labelled with lower case letters to facilitate pairing.

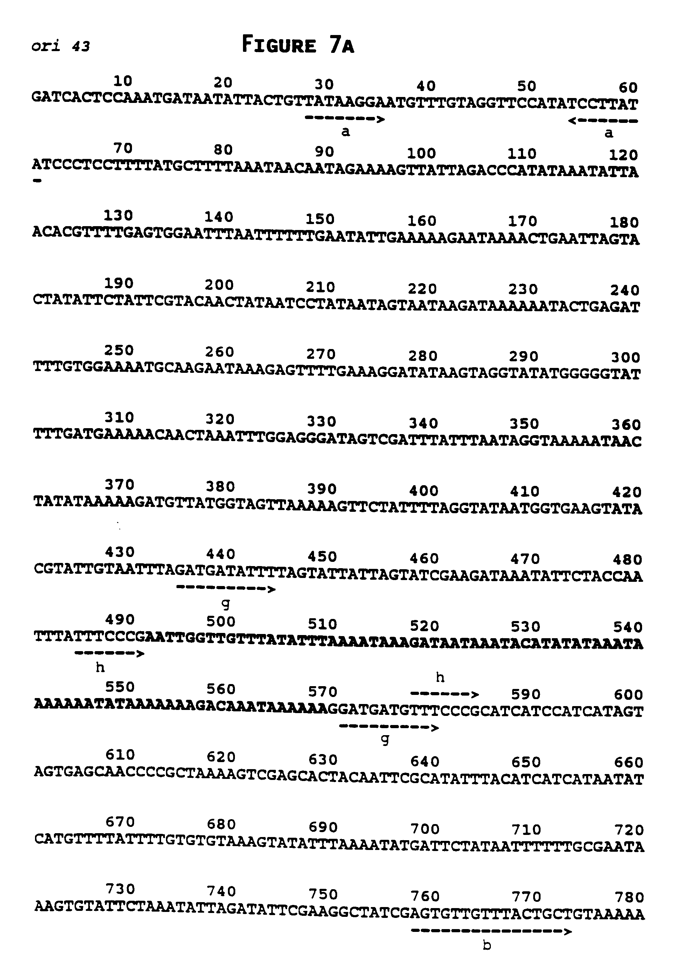

Figure 6 consists of Figures 6A, 6B and 6C and depicts the nucleotide sequence for *ori 60*, the *Bt* origin of replication derived from the 60 mDa plasmid of *Bt* var. *kurstaki* strain HD-263. The deduced amino acid sequence for the open reading frame extending from nucleotide position 627 to 1844 is also shown. DNA sequences rich in AT content (>85% AT) are indicated in boldface type. Direct and inverted repeat sequences are indicated by arrows and labelled with lower case letters to facilitate pairing.

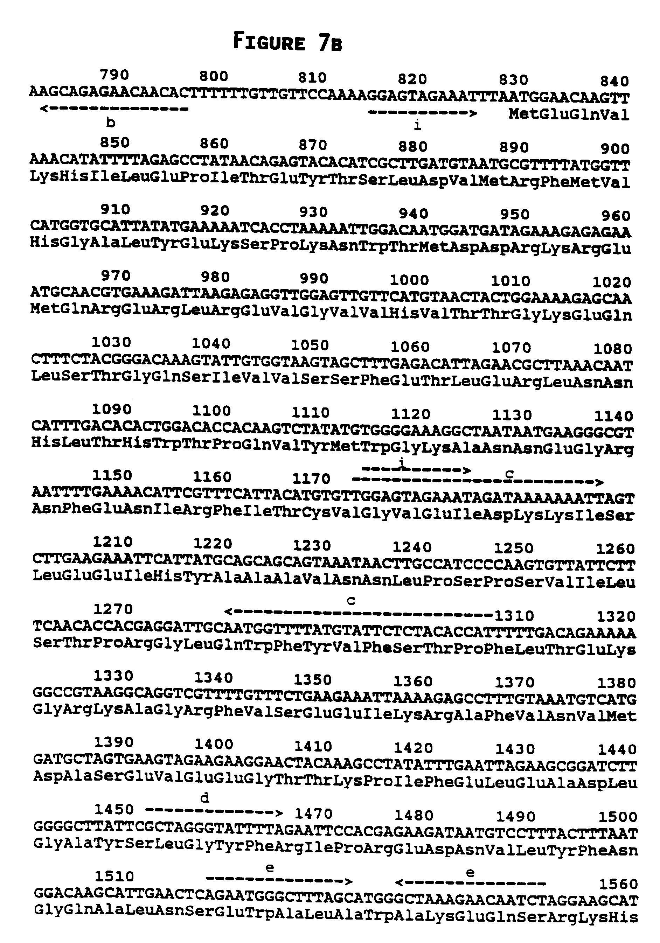

Figure 7 consists of Figures 7A, 7B, 7C and 7D and depicts the nucleotide sequence for *ori 43*, the *Bt* origin of replication derived from the 43 mDa plasmid of *Bt* var. *kurstaki* strain HD-263. The deduced amino acid sequence for the open reading frame extending from nucleotide position 829 to 2358 is also shown. DNA sequences rich in AT content (>85% AT) are indicated in boldface type. Direct and inverted repeat sequences are indicated by arrows and labelled with lower case letters to facilitate pairing.

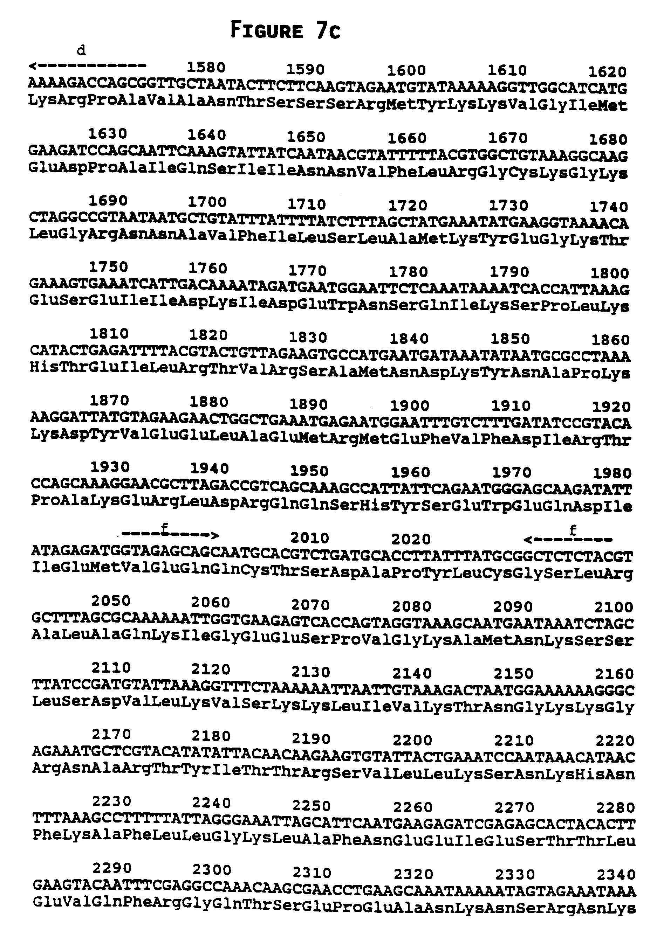

Figure 8 is a linear structural map of *ori 44* that corresponds to the nucleotide sequence shown in Figure 5. The shaded segment and its associated arrow indicate the open reading frame and direction of transcription of the replication origin gene. AT-rich sequences identified in Figure 5 are denoted in Figure 8 by the solid segments. The strategy used to determine the nucleotide sequence in *ori 44* is depicted by the horizontal arrows below the structural map. Letter abbreviations for the restriction endonuclease cleavage sites indicated in the structural map may be deciphered as follows: A=*Acc*I, Bg=*Bgl*II, Cs=*Csp*45I, E=*Eco*RI, H=*Hind*III, Nd=*Nde*I, S=*Sal*I, Xb=*Xba*I. The "250 bp" symbol is a 250 base pair length standard.

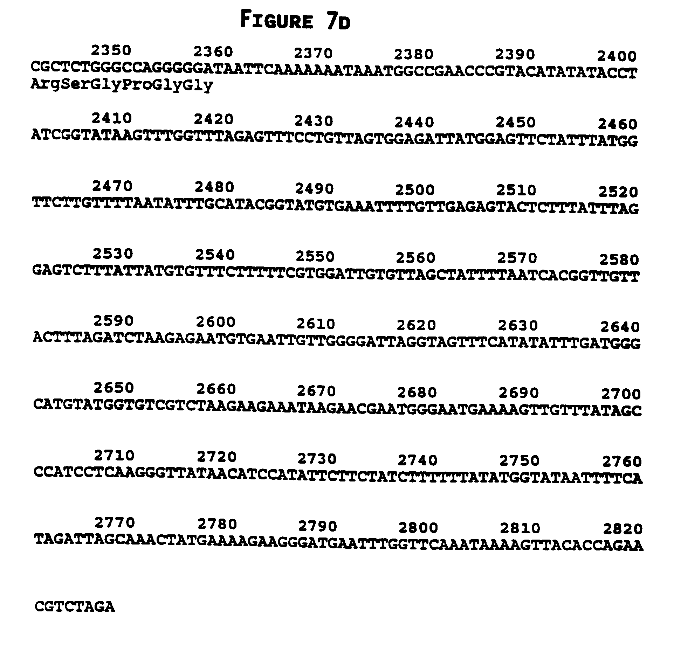

Figure 9 is a linear structural map of *ori 60* that corresponds to the nucleotide sequence shown in Figure 6. The shaded segment and its associated arrow indicate the open reading frame and direction of transcription of the replication origin gene. AT-rich sequences identified in Figure 6 are denoted in Figure 9 by the solid segments. The strategy used to determine the nucleotide sequence in *ori 60* is depicted by the horizontal arrows below the structural map. Letter abbreviations for restriction sites in the structural map are deciphered in the Figure 8 summary. The "500 bp" symbol is a 500 base pair length standard.

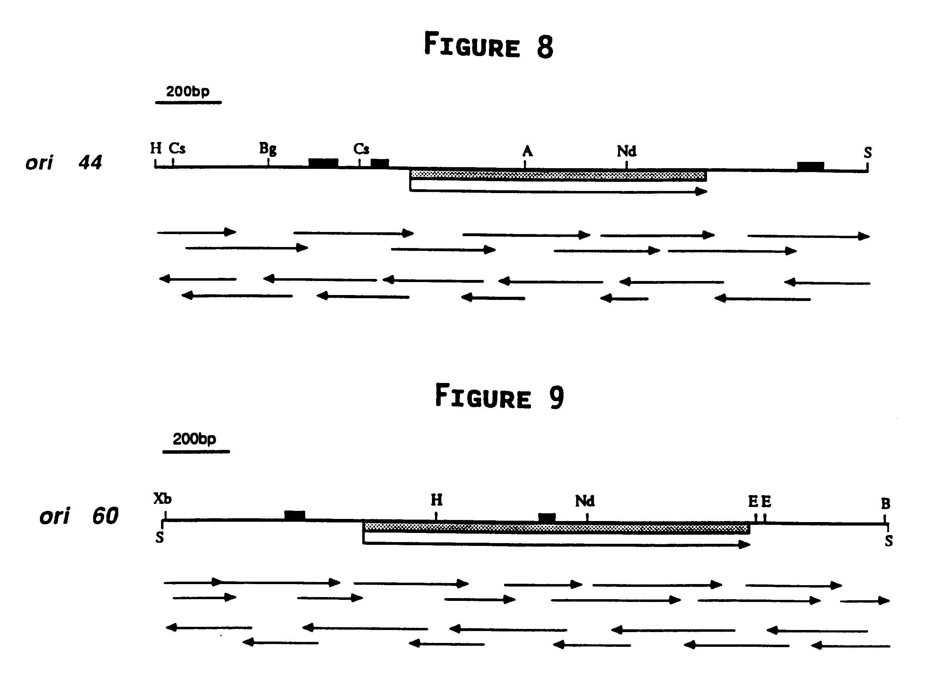

Figure 10 is a linear structural map of *ori 43* that corresponds to the nucleotide sequence shown in Figure 7. The shaded segment and its associated arrow indicate the open reading frame and direction of transcription of the replication origin gene. The AT-rich sequence identified in Figure 7 is denoted in Figure 10 by the solid segment. The strategy used to determine the nucleotide sequence in *ori 43* is depicted by the horizontal arrows below the structural map. Letter abbreviations for restriction sites in the structural map are deciphered in the Figure 8 summary. The "250 bp" symbol is a 250 base pair length standard.

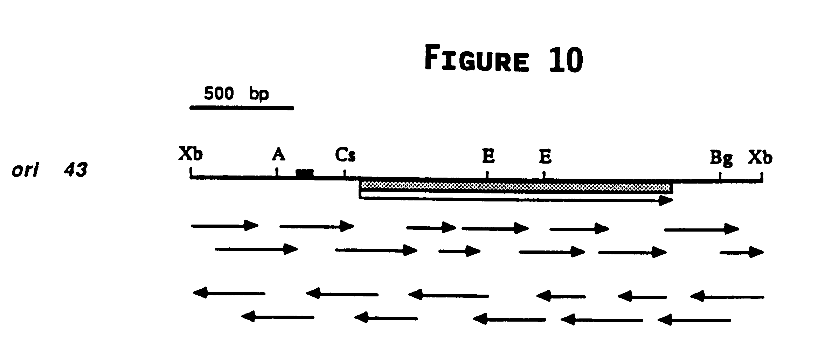

Figure 11 is a schematic diagram illustrating the construction of plasmid shuttle vector pEG597, containing the *Bt*-derived origin of replication *ori 44*. Plasmid shuttle vector pEG597 was derived from plasmid pEG851 (see Figure 3A), which also carries *ori 44*, by the sequence of steps shown in Figure 11. In the first step an *Sph*I (Sp) site was removed from the *cat* segment, by digesting pEG851 with *Sph*I, rendering the DNA ends blunt with T4 DNA polymerase and deoxynucleotide triphosphates (dNTPs), and thereafter ligating the blunt ends with T4 DNA ligase to yield the resultant plasmid as shown. In the next step, a *Not*I (N) site was inserted at an *Eco*RI (E) site, as shown. A multiple cloning site (MCS) was then inserted at a *Hind*III (H) site, to yield the plasmid shuttle vector pEG597. The linear structural maps for pEG851 and pEG597 utilize the same abbreviations that are deciphered and explained in the summaries for Figure 3 and Figure 1, respectively.

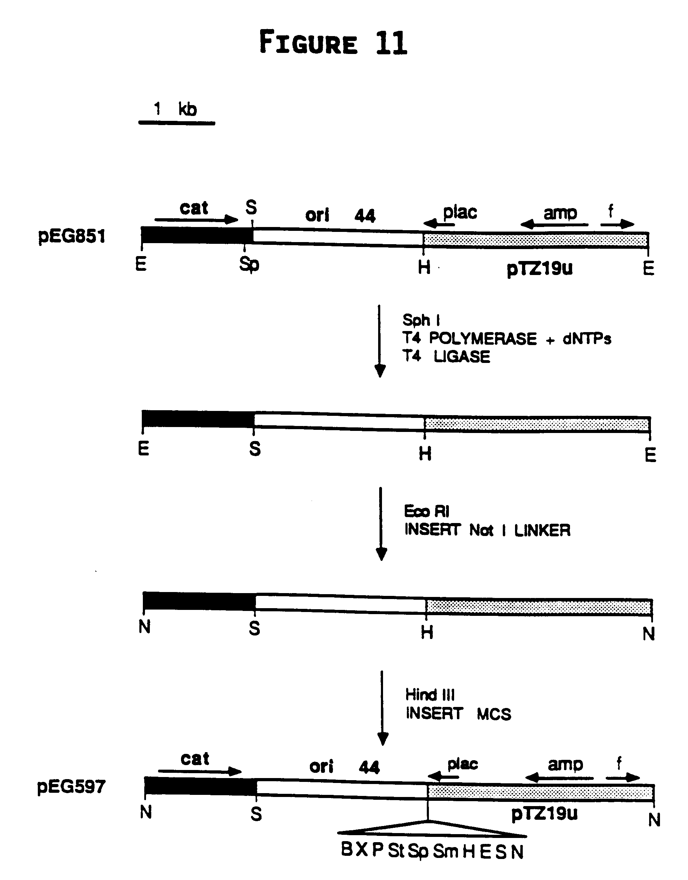

Figure 12 is a schematic diagram illustrating the construction of plasmid shuttle vectors pEG853, containing the *Bt*-derived origin of replication *ori 60*, and pEG854, containing the *Bt*-derived origin of replication *ori 43*. Both of these plasmid shuttle vectors were derived from plasmid shuttle vector pEG597 (see Figures 1 and 11) by the sequence of steps depicted. In the first step, a 2.3 kb *Sal*I fragment carrying *ori 60* from pEG588-14a (see Figure 3B) was ligated to a 4.35 kb pTZ19u-*cat Sal*I fragment from pEG597 (from which *ori 44* had been deleted), to yield plasmid pEG852. In the next two steps, an *Sfi*I (Sf) site was inserted at the *Xba*I (Xb) site of pEG852 and then a multiple cloning site (MCS) was inserted at the *Bam*HI (B) site, to make plasmid shuttle vector pEG853. Removal of the *ori 60* replication origin from pEG853 by *Xba*I digestion and insertion of a 2.8 kb *Xba*I fragment carrying *ori 43* from pEG599 (see Figure 3C) yielded plasmid shuttle vector pEG854, as shown in the last step. The linear structural maps for pEG597, pEG853 and pEG854, and for their related intermediate plasmids shown in Figure 12, utilize the same abbreviations that are deciphered and explained in the summary for Figure 1.

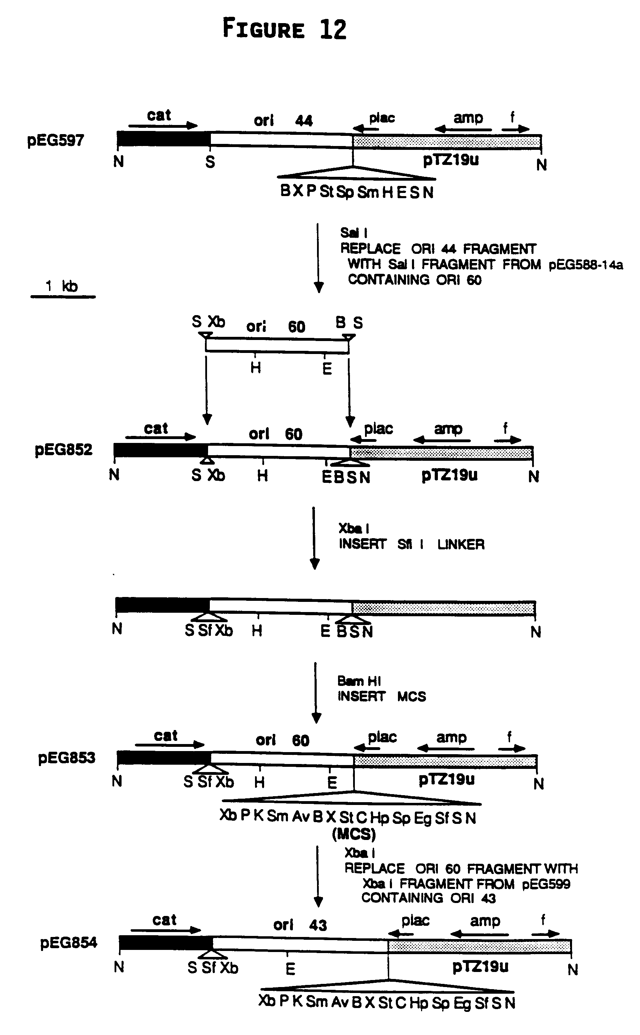

Figure 13 is a photograph of a Coomassie-stained SDS-polyacrylamide gel that shows bands of proteins synthesized by recombinant *Bt* strains derived from *Bt* var. *kurstaki* strain HD73-26. Each of these recombinant *Bt* strains contains a plasmid shuttle vector of this invention which also contains a *Bt* toxin gene, either *cryIA(c)* or *cryIIA*, as indicated in the Figure. Cultures of these *Bt* strains were grown in a liquid growth medium either in the presence (+) of 5 µg/ml chloramphenicol (Cm) or in the absence (-) of this antibiotic, as shown in the Figure. Purified CryIA(c) toxin protein (a P1-type protein) and purified CryIIA toxin protein (a P2 protein) are both included as protein standards. The nonrecombinant acrystalliferous *Bt* strain HD73-26, which contained no plasmids other than its native cryptic 4.9 mDa plasmid, was used as a control.

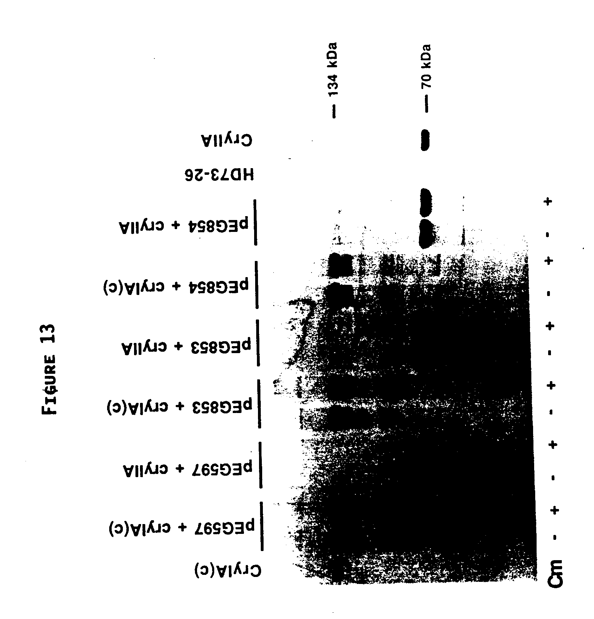

Figure 14 consists of Figures 14A and 14B. Figure 14A is a photograph of a Coomassie-stained SDS-polyacrylamide gel showing crystal proteins made by a recombinant *Bt* var. *aizawai* strain EG6346 containing pEG863 (plasmid shuttle vector pEG597 containing a *cryIA(c)* toxin gene). Two *Bt* strain controls were used: *Bt* var. *aizawai* EG6345 and its cured derivative, *Bt* strain EG6346; both strains produce crystal protein active against *Spodoptera exigua*. Purified CryIA(c) toxin protein was also included as a protein standard, as shown. Three toxin protein bands are evident in crystal preparations from *Bt* strain EG6345, while two toxin protein bands are evident in crystal preparations from *Bt* strain EG6346. Figure 14B is a photograph of a Western blot from the gel shown in Figure 14A, using antibodies specific for CryIA(c) toxin protein (a P1-type protein) to demonstrate expression of the *cryIA(c)* gene in *Bt* strain EG6346/pEG863.

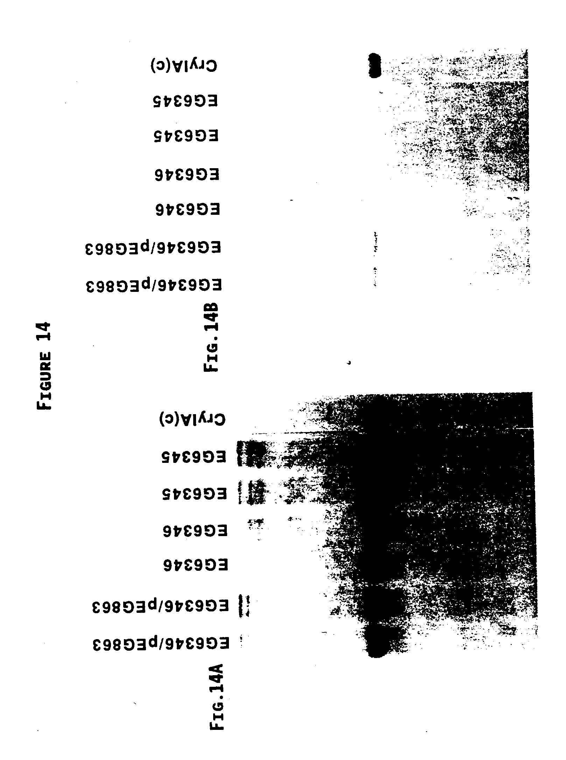

Figure 15 depicts bioassay results (shown in Table 4) for three *Bt* strains tested for insecticidal activity against six lepidopteran insect species. The *Bt* strains were *Bt* strain EG6346 containing the toxin-encoding plasmid shuttle vector pEG863, and native *Bt* strain EG6345 and its cured derivative EG6346 as controls. Insecticidal activity is measured as LC50 values, in nanoliters/cup of spore/crystal preparation required to kill 50% of the insect larvae. The insect species tested were *Spodoptera exigua* (SE), *Ostrinia nubilalis* (ON), *Heliothis virescens* (HV), *Heliothis zea* (HZ), *Trichoplusia ni* (TN) and *Plutella xylostella* (PX).

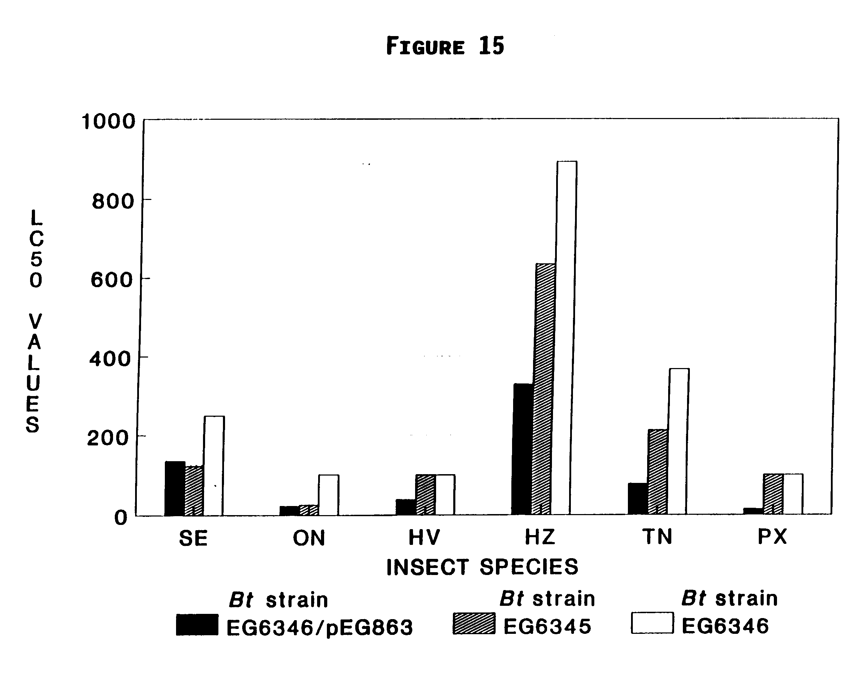

Figure 16 shows linear structural maps for two marker plasmids, pEG872 and pEG871, that contain a *cryIIIA-lacZ* gene fusion and that are otherwise identical except for their *Bt* origins of replication. The marker plasmids pEG872 and pEG871 were derived from plasmid shuttle vectors pEG854 and pEG853, respectively (see Figure 1), by insertion into their respective multiple cloning sites of a *Bam*HI-*Sph*I restriction fragment containing the *cryIIIA-lacZ* gene fusion which also contains a short segment of DNA from pBR322 (cross hatched segment). The source or identity of the various DNA segments in the plasmids is indicated by the marked boxes below the structural maps. Abbreviations: p*cryIIIA*=*cryIIIA* gene promoter fragment; *lacZ*=*lacZ* gene; 1 kb=1,000 base pair length standard. Other abbreviations, including letter abbreviations for the restriction sites, are deciphered and explained in the summary for Figure 1.

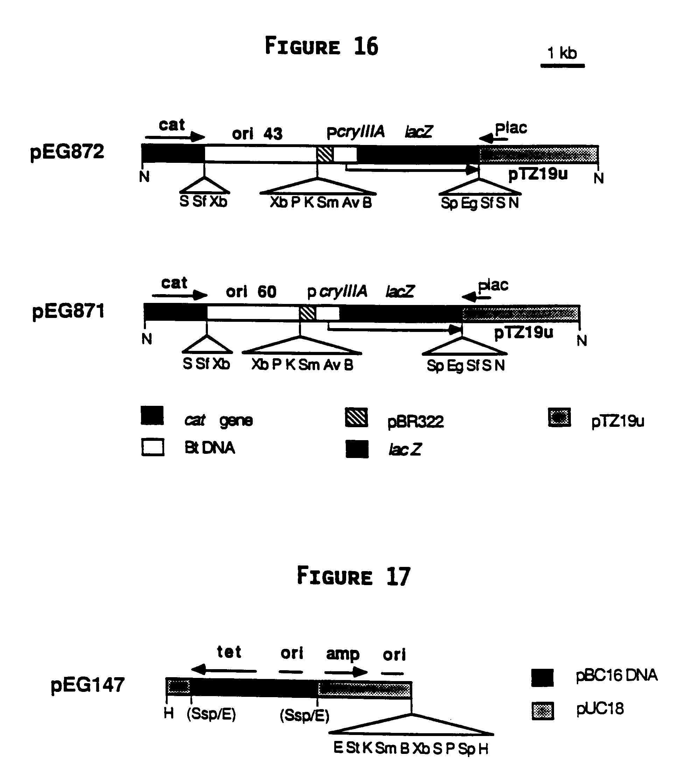

Figure 17 is a linear structural map for the plasmid shuttle vector pEG147. Plasmid pEG147 was derived by insertion of an *Eco*RI fragment from the well-known plasmid pBC16 (solid segment) into the *Ssp*I site (Ssp) of the well-known *E.coli* cloning vector pUC18 (shaded box). The pBC16-derived portion of pEG147 contains a replication origin (ori) from pBC16 that is functional in *Bt* and that is compatible with the *Bt*-derived replication origins *ori 43*, *ori 60* and *ori 44*. The tetracycline antibiotic resistance marker gene (tet) is also functional in *Bt*. The pUC18-derived segment of pEG147 contains an origin of replication (ori) functional in *E.coli* and an ampicillin antibiotic resistance marker gene (amp). The multiple cloning site shown by the triangular segment is part of pUC18. Letter abbreviations for the restriction sites indicated in the structural map are deciphered in the summary for Figure 1.

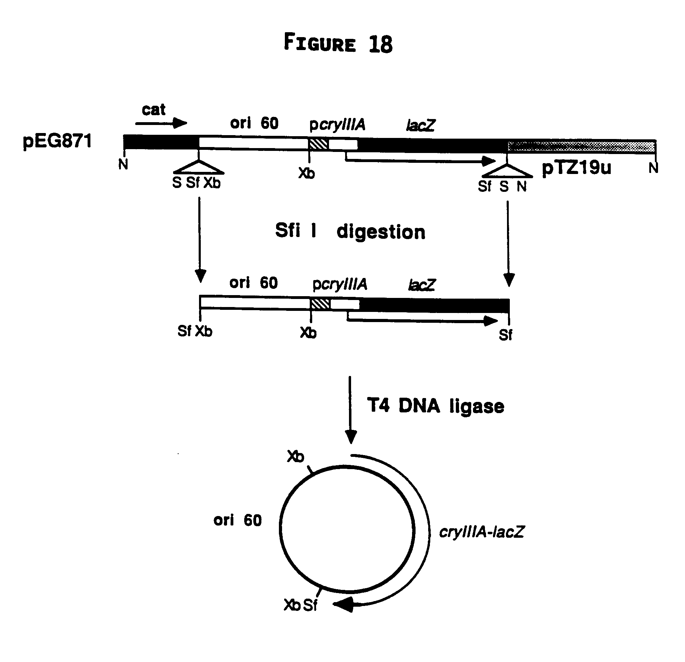

Figure 18 includes a linear structural map of the plasmid shuttle vector pEG871 (also shown in Figure 16) and illustrates digestion of pEG871 with the restriction endonuclease *Sfi*I (Sf) to recover a DNA fragment from pEG871 that contains the *Bt* replication origin *ori 60* and a *lacZ* gene with an associated *cryIIIA* promoter. The *Sfi*I restriction fragment may then be self-ligated with T4 DNA ligase, as shown in the Figure, and introduced into a *Bt* host via cotransformation, as described in Example 10. Abbreviations and symbols are the same as those deciphered and explained in the summary for Figure 16.

### Description of the Preferred Embodiments

The novel plasmid shuttle vector of this invention is a bifunctional recombinant DNA cloning vector that is intended for use in *Bacillus* and *E.coli* microorganisms. The vector is designed to permit the introduction of cloned *Bt* insecticidal toxin genes, or other genes encoding insecticidal products, into *Bt* resulting in recombinant *Bt* strains that are essentially *Bt*-derived and that contain no DNA from non-*Bt* biological sources. Recombinant *Bt* strains obtained via use of the shuttle vector of this invention are attractive from an environmental release standpoint since they contain recombinant toxin-encoding plasmids whose DNA content is essentially *Bt*-derived.

The plasmid shuttle vector contains a functional *Bt* origin of replication, a functional *E.coli* origin of replication, and optionally one or more deletable DNA segments that confer resistance to antibiotics. The vector also contains a synthetic multiple cloning site and one or more pairs of unique restriction endonuclease cleavage sites that permit various adaptations of the vector to be made.

The unique restriction sites in the shuttle vector allow: 1) excision of a single DNA fragment from the cloning vector that contains the *Bt* origin of replication, the multiple cloning site, and any gene cloned into the multiple cloning site, the excised DNA fragment being suitable for self-ligation and introduction into *Bt* as a functional, non-selectable plasmid via cotransformation of *Bt* with a selectable plasmid; 2) replacement of the *Bt* origin of replication with an alternative origin of replication, preferably a *Bt* replication origin; and 3) deletion of the *E.coli* origin of replication or replacement of the *E.coli* replication origin with an alternative origin of replication. These characteristics of the plasmid shuttle vector make it extremely useful in the development of recombinant strains of *Bt* as biopesticides.

Two adjacent elements in the shuttle vector, the *Bt*-derived origin of replication and the multiple cloning site, are flanked by unique restriction sites. These restriction endonuclease cleavage sites should be unique to the plasmid shuttle vector and absent from the *Bt* toxin-encoding gene fragment that is to be cloned and inserted into a recombinant *Bt* strain.

The "unique" restriction endonuclease cleavage sites referred to in this specification may be selected using either or both of two criteria: (i) 8 base pair restriction sites are generally more rare than 6 base pair sites; (ii) the *Bt* genome is estimated to be only 30% GC so GC-rich restriction sites, e.g., *Sfi*I and *Not*I, are exceptionally rare.

The unique flanking sites surrounding the *Bt* replication origin and multiple cloning site are desirably *Sfi*I and/or *Sal*I restriction sites. Because these restriction sites, particularly *Sfi*I, occur infrequently in the *Bt* genome (and therefore rarely found in *Bt* toxin genes of interest), they provide a reliable means of recovering a single DNA fragment from the plasmid shuttle vector that contains the *Bt* replication origin and any *Bt* toxin gene cloned into the multiple cloning site. The recovered single DNA fragment containing both the *Bt* replication origin and the multiple cloning site is readily self-ligated by conventional methods, e.g., using T4 DNA ligase to join the fragment termini. The ability to combine a cloned *Bt* toxin gene and a *Bt* origin of replication on a self-ligatable fragment of DNA, via the plasmid shuttle vector of this invention, provides a means of introducing small, toxin-encoding functional plasmids into a *Bt* host via cotransformation.

The flanking sites surrounding the *Bt* replication origin and multiple cloning site are preferably identical but may alternatively be compatible with each other. There may also be two sets of such flanking sites, e.g., one flanking pair being *Sfi*I sites and the other flanking pair being *Sal*I sites.

The DNA restriction fragments containing the origin of replication functional in *E.coli* and the *Bt*-derived origin of replication are each removable from the plasmid shuttle vector. The unique restriction sites that flank the DNA fragment containing the *E.coli* replication origin are desirably identical, but may alternatively be compatible with each other. The rare restriction site *Not*I is preferably used for the sites flanking or surrounding the *E.coli* DNA segment, since the *Not*I site occurs infrequently in the *Bt* genome (and therefore are rarely found in *Bt* toxin genes of interest).

Similarly, the restriction sites that flank the *Bt*-derived origin of replication, or the DNA fragment containing the *Bt*-derived replication origin, are desirably unique to the plasmid shuttle vector and are preferably identical, but may alternatively be compatible. The restriction endonuclease cleavage site *Xba*I is preferred for this purpose.

These unique restriction sites make it possible to replace the respective *E.coli* and *Bt* origins of replication with alternative origins of replication, thereby facilitating the construction of additional cloning vectors that may offer advantages over the specific plasmid shuttle vectors exemplified in this disclosure. For example, additional *Bt* replication origin clones may be found that exhibit increased stability over those presently disclosed in this specification. Numerous plasmids containing different replication origins can be detected among the many varieties of *Bt*. Any of these plasmids can likely serve as the source for additional, newly characterized *Bt* replication origins employing the cloning procedure outlined below. Such new *Bt* replication origins can be readily inserted into the plasmid shuttle vectors pEG853 and pEG854, depicted in Figure 1 and described in detail below, for further testing.

Other advantages to the removable nature of the *Bt* and *E.coli* origins of replication in the plasmid shuttle vector should be apparent to those skilled in the art. For fermentation research with a recombinant *Bt* strain, the DNA fragment with the *E.coli* origin of replication can be deleted from an appropriate plasmid shuttle vector to yield a toxin-encoding plasmid that consists of a *Bt* replication origin, a *Bt* toxin gene inserted into the multiple cloning site, and a selectable antibiotic resistance gene functional in *Bt*. The resultant plasmid, composed primarily of *Bt*-derived DNA, serves as an intermediate construct whose characteristics can be readily evaluated in the absence of *E.coli* DNA in scaled-up fermentation experiments. Such characteristics include its segregational stability in *Bt*, its impact on overall crystal toxin production in recombinant *Bt* strains, and its effect on the insecticidal activity of recombinant *Bt* strains. The stability of such recombinant plasmids is described in greater detail in Example 7.

The plasmid shuttle vector of this invention exploits the discovery that small plasmids, i.e., those significantly smaller than 30 mDa, that utilize native *Bt* plasmid replication origins and that contain *Bt* toxin genes, can be stably maintained in *Bt* host strains without selection. The available literature on *Bt* strain characteristics teaches that *Bt* plasmids harboring toxin genes are virtually always larger than 30 mDa, even though no reasons are given why this should be the case. The absence of small toxin-encoding plasmids in *Bt* is an enigma, particularly since small cryptic plasmids, i.e., plasmids whose encoded gene products are unknown, are abundant in *Bt*. Using the plasmid shuttle vectors described in this specification, whose *Bt* replication origins were derived from large *Bt* plasmids, two different *Bt* toxin genes were introduced into a *Bt* strain via electroporation and the recombinant plasmids shown to be stably maintained in such *Bt* constructs despite the relatively small size of the plasmids. The details of this work are presented in Examples 6 and 7.

The novel shuttle vectors of this invention contain an origin of replication derived from a *Bt* plasmid. The isolation and characterization of *Bt* replication origins and the DNA sequence analysis of three *Bt* replication origins were based on the inventor's studies with plasmid-cured derivatives of two well-known *Bt* strains, *Bt* var. *kurstaki* HD-263 and *Bt* var. *kurstaki* HD-73 (see Gonzalez, Jr. et al., *Proc.Natl.Acad.Sci.USA*, 79, pp.6951-6955 (1982)). The cured derivatives of these two strains were *Bt* strain HD263-6, *Bt* strain HD73-26, and *Bt* strain HD73-26-10, the latter being a transconjugant strain of the cured derivative HD73-26 containing a 44 mDa plasmid from *Bt* strain HD-263. It should be evident from the procedures described below that other *Bt* strains could have been used as the source material for obtaining *Bt* origins of replication for use in this invention. A general description of the methodology used in these procedures follows.

Electroporation was used to clone *Bt* plasmid replication origins directly in *Bt*, using a cloning vector, pEG588, which lacks a *Bt*-functional replication origin but contains a chloramphenicol antibiotic resistance gene, *cat*, that is functional in *Bt*. Insertion of DNA fragments containing *Bt* replication origin(s) into the pEG588 vector enabled it to transform *Bt* to chloramphenicol resistance.

The resident plasmids in *Bt* strains HD73-26-10 and HD263-6 were utilized for the replication origin cloning procedure, but any *Bt* strain containing plasmids, either toxin-encoding or cryptic, could be used instead. *Bt* strain HD73-26-10 is a transconjugant derivative of *Bt* strain HD73-26 that contains plasmids of 44 and 4.9 mDa; the 44 mDa plasmid carries a *cryIA(c)* gene. *Bt* strain HD263-6 contains plasmids of -130, -110, 60, 43, 7.5, 5.4, 5.2, and 4.9 mDa; toxin genes are located on the approximately 110 and 60 mDa plasmids. Plasmid DNA from *Bt* strains HD263-6 and HD73-26-10 was isolated, partially digested with the restriction endonuclease *Mbo*I, and ligated into the *Bam*HI site of pEG588. The ligation products were used to transform the acrystalliferous *Bt* strain HD73-26 to chloramphenicol resistance. Chloramphenicol-resistant *Bt* clones harboring novel plasmids were characterized by Southern blot analysis and the plasmids grouped according to their replication origins. The clones from *Bt* strain HD263-6 contained replication origins from the 60, 43, 7.5, 5.4, 5.2 and 4.9 mDa plasmids of *Bt* strain HD263-6 while the *Bt* strain HD73-26-10 clones contained replication origins from the 44 and 4.9 mDa plasmids of *Bt* strain HD73-26-10.

The *Bt* replication origin from the 60 mDa plasmid was contained on a 2.3 kilobase (kb) DNA fragment. The *Bt* replication origins from the 44 and 43 mDa plasmids were readily localized to small DNA fragments of 2.2 and 2.8 kb, respectively, as described in the Examples. These three *Bt* replication origins exhibited good segregational stability in the absence of selection in transformed acrystalliferous *Bt* strain HD73-26 and thus were selected as being suitable for use in subsequent cloning vector development.

The three *Bt* replication origins were further characterized by DNA sequence analysis. Briefly, the cloned replication origins were inserted in the well-known M13 phage vectors mp18 and/or mp19 and the recombinant phages propagated in the *E.coli* host TG1. Standard techniques for DNA sequence analysis of the single-stranded DNA templates were employed. Surprisingly, the sequence analysis revealed that the three *Bt* replication origins, though derived from plasmids of similar size (43, 44 and 60 mDa) of which two encode toxin genes (44 and 60 mDa), showed no significant homology to each other, either at the nucleotide or amino acid sequence level. These results, taken together with the Southern blot data, discussed in Example 2 and shown in Figure 4, suggest that the replication origins of *Bt* plasmids may individually be unique, rather than be related to each other. Furthermore, comparisons with published sequences for replication origins isolated from other gram-positive bacteria indicate no significant homology with the sequences of the three *Bt* replication origins disclosed here, either at the nucleotide or amino acid sequence level.

The shuttle vector of this invention also contains a removable DNA fragment with an origin of replication functional in *E.coli.* In this aspect of the shuttle vector design, the Examples describe the placement of a rare restriction site (*Not*I) at each end of a DNA segment from the vector which contains an origin of replication functional in *E.coli*. This construction enables the *E.coli*-derived DNA fragments to be deleted from the shuttle vector when desired.

The shuttle vector may optionally contain antibiotic resistance genes, and these are intended to be readily removable. In such cases and as described in the Examples, *Sfi*I and/or *Sal*I sites may be placed at each end of the DNA segment containing the *Bt* origin of replication and adjacent multiple cloning site. This makes it possible to recover from the shuttle vector self-ligatable DNA fragments consisting of a *Bt* replication origin and a multiple cloning site, optionally with an inserted, cloned toxin gene. In this manner, non-*Bt* DNA sequences originally present in the shuttle vector may be excised, e.g., the *E.coli*-functional replication origin and its adjacent, optional antibiotic resistance marker gene.

Another feature of the shuttle vector of this invention is that the *Bt* replication origin is replaceable with other suitable *Bt* replication origins. Two of the exemplified shuttle vectors of this invention (pEG853 and pEG854) contain *Xba*I sites at each end of the *Bt* replication origin fragment to permit the substitution of other replication origins.

The multiple cloning site is another element of the shuttle vector. Construction of multiple cloning sites, sometimes referred to as polylinkers, is well-known to those skilled in the art and thus does not require further detailed description. In the Examples described below, synthetic oligonucleotides generated on an Applied Biosystems 380B DNA Synthesizer were used to introduce new restriction endonuclease sites and multiple cloning sites into the vectors, using procedures well known to those skilled in the art.

One preferred multiple cloning site for use in the plasmid shuttle vector of this invention has restriction sites in the following sequence, with the *Xba*I site being located adjacent to the *Bt*-derived origin of replication; *Xba*I *Pst*I *Kpn*I *Sma*I *Avr*II *Bam*HI *Xho*I *Sst*I *Cla*I *Hpa*I *Sph*I *Eag*I *Sfi*I *Sal*I *Not*I. The nucleotide sequence for this multiple cloning site is as follows:

(5')
TCTAGACTGCAGGTACCCGGGCCTAGGATCCCTCGAGCTCATCGATGTTAACGCATGCGGCCGATCGG
GCCGATCCGTCGACGCGGCCGC (3').

A second preferred multiple cloning site has restriction sites in the following sequence, with the *Bam*HI site being located adjacent to the *Bt*-derived origin of replication: *Bam*HI *Xho*I *Pst*I *Sst*I *Sph*I *Sma*I *Hind*III *Eco*RI *Sal*I *Not*I. The nucleotide sequence for this second multiple cloning site is as follows: 
(5')
GGATCCCTCGAGCTGCAGGAGCTCGCATGCCCCGGGAAGCTTGAATTCGTCGACGCGGCCGC (3').
Three different plasmid shuttle vectors, which may also be called cloning vectors, are exemplified in this disclosure. The shuttle vector containing the *Bt* replication origin from the 44 mDa plasmid is designated pEG597. The shuttle vector containing the *Bt* replication origin from the 60 mDa plasmid is designated pEG853. The shuttle vector containing the *Bt* replication origin from the 43 mDa plasmid is designated pEG854.

The toxin genes *cryIA(c)* and *cryIIA*, both cloned from resident plasmids of *Bt* strain HD-263, were inserted into the three shuttle vectors, as described in more detail in Example 6. The toxin-encoding plasmids were then introduced into the acrystalliferous *Bt* strain HD73-26 via electroporation, and the resultant recombinant *Bt* strains were subsequently assessed for toxin production and plasmid stability. Recombinant *Bt* strains harboring the *cryIA(c)* gene produced significant levels of CryIA(c) toxin protein; likewise, recombinants harboring the *cryIIA* gene produced significant levels of CryIIA toxin protein. The PEG853 and pEG854-based plasmids exhibited excellent segregational stability in the absence of selection with chloramphenicol, despite their relatively small size. Small plasmids harboring insecticidal toxin genes are conspicuously absent from native *Bt* strains, so the stability observed for the recombinant pEG853- and pEG854-based plasmids was not expected.

As a demonstration of the utility of the shuttle vectors of this invention for *Bt* recombinant strain development, the plasmid pEG863 carrying the *cryIA(c)* gene was introduced into a *Bt* strain, *Bt* var. *aizawai* strain EG6346, that exhibits relatively good insecticidal activity against *Spodoptera exigua* (beet armyworm) but commercially-unacceptable activity against a variety of other lepidopteran insect pests, including *Plutella xylostella* (diamondback moth) and *Trichoplusia ni* (cabbage looper). The recombinant *Bt* strain EG6346, containing pEG863 with its inserted *cryIA(c)* gene, produced the CryIA(c) toxin protein and exhibited good insecticidal activity against all three insect pests, due to the potent insecticidal activity of the CryIA(c) toxin protein against *Plutella xylostella* and *Trichoplusia ni.* This is described in more detail in Example 8.

Electroporation studies were conducted to confirm the feasibility of using cotransformation to introduce self-ligated plasmid DNA's lacking selectable marker genes into *Bt*, as described in Example 9. For this work, a DNA fragment containing a *cryIIIA*-*lacZ* gene fusion (the first moiety containing a promoter from a coleopteran-active *Bt* toxin gene) was inserted into the multiple cloning site of shuttle vectors pEG853 and pEG854 to yield plasmids pEG871 and pEG872, respectively. The *cryIIIA-lacZ* gene encodes a fusion protein with beta-galactosidase activity. This enzymatic activity may be used to convert the chromogenic dye indicator X-Gal (5-bromo-4-chloro-3-indolyl β-D-galactopyranoside) to a blue pigment. *Bt* colonies expressing the *cryIIIA-lacZ* gene protein product are blue in color when grown on plates containing X-Gal.

In preparation for cotransformation, the *cryIIIA-lacZ* plasmids pEG871 and pEG872 were cleaved with the restriction endonucleases *Sfi*I and *Not*I, ligated with T4 DNA ligase, and combined with pEG147 (a plasmid containing a tetracycline resistance gene, *tet*) to transform the acrystalliferous *Bt* strain HD73-26 to tetracycline resistance by electroporation. Self-ligation of the *Sfi*I fragments of pEG871 and pEG872 containing *cryIIIA-lacZ* and a *Bt* replication origin yielded non-selectable plasmid DNAs (i.e., ones not containing the selectable cat gene) capable of replicating in *Bt*. The electroporated cells were spread onto agar plates containing tetracycline and X-Gal and grown overnight at 30°C. About 2-5% of the tetracycline-resistant *Bt* colonies were blue and chloramphenicol-sensitive, indicating that these colonies contained the *lacZ* gene but lacked the chloramphenicol resistance gene (*cat*) and presumably lacked the *E.coli*-functional origin of replication from pEG871 and pEG872. The recovery of blue, chloramphenicol-sensitive colonies demonstrated that the self-ligated *Sfi*I fragments from pEG871 and pEG872 were successfully introduced into *Bt* by cotransformation, resulting in recombinant plasmids that contained the *cryIIIA-lacZ* gene but lacked antibiotic resistance genes and an *E.coli*-functional origin of replication. Removal of pEG147, containing the tetracycline resistance gene, may be achieved by subsequently selecting for tetracycline-sensitive *Bt* colonies. The resultant tetracycline-sensitive *Bt* strain would contain only a recombinant plasmid bearing a *Bt* replication origin and the *cryIIIA-lacZ* gene inserted into the multiple cloning site.

The results of the cotransformation studies with pEG871 and pEG872 described in Example 9, together with the data demonstrating the segregational stability of small toxin-encoding plasmids in *Bt*, indicate that the present invention is useful in the development of recombinant *Bt* strains free of foreign DNA derived from biological sources.

This same cotransformation procedure can be adapted to introduce toxin genes into *Bt* via the shuttle vector of this invention, using conventional colony blot hybridization procedures and radiolabelled toxin gene-specific hybridization probes, instead of a *lacZ* marker gene, to identify among the tetracycline-resistant colonies those colonies that contain the non-selectable toxin-encoding plasmid derived from the shuttle vector. In the colony blot hybridization procedure, the tetracycline-resistant *Bt* colonies are partially transferred to a nitrocellulose filter where they are lysed and the released plasmid DNA is immobilized on the filter. Hybridization of the immobilized plasmid DNA to a radiolabelled toxin gene fragment can be detected by autoradiography, thereby enabling identification of colonies containing a toxin-encoding plasmid. Tetracycline-resistant *Bt* clones containing the recombinant toxin-encoding plasmid may be subsequently tested for growth on chloramphenicol to demonstrate absence of the chloramphenicol resistance gene, cat. As before, removal of the selectable plasmid pEG147, containing the tetracycline resistance gene, may be achieved by subsequently selecting for tetracycline-sensitive colonies. The resultant tetracycline-sensitive *Bt* strain would contain only a recombinant plasmid bearing a *Bt* replication origin and a toxin gene inserted into the multiple cloning site.

The following Examples provide further explanation of the invention and the procedures used to construct the shuttle vectors of this invention. Those familiar with the state of the art will recognize that in many instances, more than one experimental protocol can be employed to achieve a desire end. Specific procedures have been cited or described where deemed appropriate, but these should not be taken as the exclusive means by which the Examples can be carried out.

### Example 1

### Construction of a cloning vector, pEG588, useful for isolating replication origins from Bt

The plasmids found in the *Bt* strain HD-263 var. *kurstaki*, obtained from the H. Dulmage collection of *Bt* strains, were selected as the source material for the replication origin cloning procedures described in subsequent Examples. However, any *Bt* strain harboring plasmids may be used to clone plasmid replication origins. Conventional recombinant DNA techniques were employed in the replication origin cloning experiments and in construction of the vectors. The laboratory manual of Maniatis et al., "Molecular Cloning: A Laboratory Manual", 1982, Cold Spring Harbor Laboratory, Cold Spring Harbor, NY, provides a useful reference source for these techniques.

In this Example, plasmid pEG588 was constructed to serve as a cloning vector useful for recovering replication origins from *Bt*. To this end, the *E.coli* plasmid pTZ18u was digested with the restriction endonuclease *Eco*RI and ligated to a 1.5 kilobase (kb) *Eco*RI fragment from pMI1101 containing the chloramphenicol acetyltransferase (*cat*) gene of pC194 in order to provide the cloning vector with a selectable (chloramphenicol resistance) marker gene functional in *Bt*. The resultant plasmid construct containing the cat gene in the desired orientation, designated as pEG588, is shown in the structural map of Figure 2. The pEG588 cloning vector was propagated in the *E.coli* host strain GM2163, obtained from New England Biolabs, Inc.

The pEG588 plasmid cannot replicate in *Bt* because it lacks a replication origin functional in *Bt*. Subsequent Examples describe how cloned DNA fragments from two *Bt* strains were used to confer autonomous replication in *Bt* on this vector.

### Example 2

### Cloning of replication origins from Bt

The *Bt*-derived origins of replication were obtained primarily from plasmids in two *Bt* strains, *Bt* var. *kurstaki* strain HD-73 and strain HD-263. The source plasmids included both cryptic plasmids and plasmids carrying toxin-encoding genes. The latter plasmids included 44 mDa and 60 mDa plasmids from HD-263 that contain genes encoding the insecticidal CryIA(c) crystal protein.

To clone the replication origin from the 44 mDa plasmid of *Bt* strain HD-263, plasmid DNA from the *Bt* strain HD73-26-10, a transconjugant strain harboring the 44 mDa plasmid from HD-263 and a 4.9 mDa plasmid from HD-73, was isolated using the well-known procedure described by Kronstad et al., *J.Bacteriol.* (1983) *154*:419-428. Plasmid DNA from *Bt* strain HD73-26-10 was partially digested with the restriction endonuclease *Mbo*I to yield DNA fragments in the 2-15 kilobase (kb) range. An approximately equimolar concentration of this DNA was ligated to the cloning vector pEG588, described in Example 1, which had already been digested with the restriction endonuclease *Bam*HI. The products of the ligation reaction were used to transform *Bt* strain HD73-26, a cured acrystalliferous derivative of HD-73, to chloramphenicol resistance.

A total of twenty-one chloramphenicol resistant clones of *Bt* strain HD73-26 were recovered and analyzed on horizontal agarose gels for the presence of novel plasmids using a modified Eckhardt lysis procedure. The novel plasmids from each of these twenty-one clones, derived from pEG588, were designated as pEG588-1 through pEG588-21.

Among these twenty-one plasmids, pEG588-8 contained the smallest replication origin insert (-3.2 kb) as determined by agarose gel electrophoresis. A structural map of pEG588-8 is shown in Figure 3A. The replication origin insert from pEG588-8 was used as a hybridization probe for Southern blot analysis. Novel plasmids from 18 chloramphenicol-resistant clones, i.e., transformants, hybridized strongly to the pEG588-8 probe. The *Bt* replication origin from pEG588-8 was subcloned into pEG851 as shown in Figure 3A, and pEG851 was shown to hybridize to the 44 mDa plasmid in *Bt* strain HD73-26-10. This Southern blot hybridization result is shown in Figure 4, for Probe 1. This result demonstrates that pEG851 contains a replication origin derived from the 44 mDa plasmid of *Bt* strain HD-263.

The three remaining transformants contained novel plasmids that hybridized strongly on Southern blots to a hybridization probe consisting of the resident 4.9 mDa plasmid of strain *Bt* HD73-26. One of these plasmids, pEG588-2, was shown to hybridize to the 4.9 mDa plasmid of *Bt* strains HD73-26-10 and HD263-6. This Southern blot hybridization result is also shown in Figure 4. This result demonstrates that pEG588-2 contains a replication origin derived from the 4.9 mDa plasmid of BC strain HD73-26-10.

The replication origins of other plasmids from *Bt* strain HD-263 were obtained in similar fashion, using plasmid DNA isolated from *Bt* strain HD263-6, a cured derivative of HD-263 lacking the 44 mDa plasmid. A total of 24 chloramphenicol-resistant clones containing novel plasmids were obtained in this cloning procedure. The replication origin inserts present in these plasmids were found to fall into six distinct homology groups based on Southern blot analyses using inserts from several of these plasmids as hybridization probes. Subsequently, each homology group was matched with its resident *Bt* plasmid, as shown in Table 1, by Southern blot analysis of resident *Bt* plasmids using the smallest cloned replication origin insert of each homology group that had been shown to replicate in *Bt*.

A representative listing of the *Bt* plasmid replication origin clones from these studies is shown below in Table 1; the Table also includes pEG851. The Table indicates the resident plasmid source of the replication origin, as well as the insert size of the DNA restriction fragment in the plasmid replication origin clone. Figure 4 shows the Southern blot hybridization results using the clones listed in Table 1.

**Table 1**

| *Bt* plasmid replication origin clones | | |
|---|---|---|
| Clone | Resident *BT* plasmid | Insert size |
| pEG588-2 | 4.9 mDa from *Bt* HD-73 | 7.3 kb |
| pEG588-20a | 5.2 mDa from *Bt* HD-263 | 10.5 kb |
| pEG588-4a | 5.4 mDa from *Bt* HD-263 | 11.4 kb |
| pEG588-23a | 7.5 mDa from *Bt* HD-263 | 10.5 kb |
| pEG599¹ | 43 mDa from *Bt* HD-263 | 2.8 kb |
| pEG851² | 44 mDa from *Bt* HD-263 | 2.2 kb |
| pEG588-14a | 60 mDa from *Bt* HD-263 | 2.3 kb |

| | | |
|---|---|---|
| ¹ subcloned from pEG588-13a (Figure 3C) | | |
| ² subcloned from pEG588-8 (Figure 3A) | | |

The individual plasmids containing replication origins derived from the 4.9, 7.5, 43, 44, and 60 mDa plasmids, listed in Table 1, each showed homology only to the resident *Bt* plasmid from which it had been derived. The recombinant plasmid containing the replication origin from the 5.4 mDa plasmid, pEG588-4a, showed partial homology to the resident 4.9 mDa plasmid but not vice-versa. The recombinant plasmid containing the replication origin from the 5.2 mDa plasmid, pEG588-20a, showed strong homology to the resident 43 mDa plasmid, but not vice-versa. These data demonstrate that the replication origins derived from these resident *Bt* plasmids are non-homologous with each other. The respective DNA sequences of the *Bt* replication origins from the 44, 60 and 43 mDa plasmids, disclosed in this specification and shown in Figures 5, 6, and 7, respectively, indicate no nucleotide sequence homology among these three *Bt* replication origins.

### Example 3

### DNA sequence analysis of Bt replication origins

The *Bt* replication origins isolated from the 44, 60, and 43 mDa plasmids obtained from *Bt* var. *kurstaki* strain HD-263 have been designated *ori 44, ori 60*, and *ori 43,* respectively. Each of these three *Bt* replication origins has been isolated and completely sequenced. The DNA sequences for *ori 44*, *ori 60* and ori *43* are shown in Figures 5, 6 and 7 respectively.

The cloning and sequencing procedures for *ori 44*, *ori 60* and *ori 43* are as follows. *Ori 44* was subcloned into the M13 phage vectors mp18 and mp19 as a 2.2 kb *Hind*III/*Sal*I DNA fragment isolated from pEG851, which was previously described in Example 2 and is shown in Figure 3A. Figure 8 shows a structural map of the 2.2 kb *Hind*III/*Sal*I fragment containing ori 44.

*Ori 60* was subcloned into mp18 in both orientations as a 2.3 kb *Sal*I fragment isolated from pEG588-14a, shown in Figure 3B and listed in Table 1. Figure 9 shows a structural map of the 2.3 kb *Sal*I fragment containing *ori 60*.

*Ori 43* was subcloned into mp18 in both orientations as a 2.8 kb *Xba*I fragment isolated from pEG599, shown in Figure 3C and listed in Table 1. Figure 10 shows a structural map of the 2.8 kb *Xba*I fragment containing *ori 43.*

Single strand DNA templates were sequenced according to the dideoxy chain termination method using [alpha-³⁵S]dATP and the Sequenase® DNA sequencing kit, available from US Biochemical Corp. Synthetic oligonucleotides were generated on an Applied Biosystems 380B DNA Synthesizer to serve as primers for DNA sequence analysis. Sequence analysis was initiated using the M13 universal primer. Subsequently, sequencing primers were synthesized as needed based on the derived DNA sequences.

The complete nucleotide sequences of the *Bt* DNA fragments harboring the *Bt* replication origins *ori 44, ori 60* and *ori 43* are shown in Figures 5, 6, and 7 respectively. Linear restriction map diagrams of the replication origin fragments are shown in Figures 8 9, and 10 for *ori 44*, *ori 60* and *ori 43,* respectively.

The replication origin from the 44 mDa plasmid*, ori 44* (Figure 5), is contained on a 2249 bp DNA fragment characterized by an open reading frame (ORF) of 936 bp (312 amino acids) and an overall AT content of 71.4%. The ORF starts at nucleotide position 797 with a GUG as the probable initiation codon. The deduced protein gene product has a molecular mass of 36,478 daltons (Da). The sequence also contains segments of AT-rich DNA (>85% AT) which lie outside the ORF. Numerous direct and inverted repeats are present in the region upstream of the ORF. One of these repeats (labelled "d" in Figure 5) also spans the putative Shine-Dalgarno sequence. The ORE is followed by an inverted repeat sequence, labelled "e" in Figure 5, that could serve as a transcriptional terminator.

The replication origin from the 60 mDa plasmid, *ori 60*, (Figure 6) is contained on a 2290 bp DNA fragment characterized by an ORF of 1218 bp (406 amino acids) and an overall AT content of 69.1%. The ORF starts at nucleotide position 627 and encodes a deduced protein of 47,780 Da. The sequence is characterized by two AT-rich domains, one before and one within the ORF. The former lies among a number of direct and inverted repeats while the latter lies adjacent to a complex series of repeated sequences within the ORF. In addition to these repeats, the ORF is followed by another large inverted repeat, labelled "e" in Figure 6, that could function as a transcriptional terminator.

The replication origin from the 43 mDa plasmid, *ori 43*, (Figure 7) is contained on a 2828 bp DNA fragment characterized by an ORF of 1530 bp (510 amino acids) and an overall AT content of 67.9%. The ORF starts at nucleotide position 829 and encodes a deduced protein of 58,320 Da. As with the other two replicons, *ori 43* contains an AT-rich domain upstream of the ORF. A large inverted repeat, labelled "b" in Figure 7, is centered 51 bp upstream of the putative initiative condon. One copy of an 11 bp direct repeat, labelled "i", lies over the putative Shine-Dalgarno sequence. The other copy, which begins at nucleotide 1174, comprises part of a 21/26 bp inverted repeat, labelled "c" in Figure 7, within the ORF. In contrast to the other two replication origins, an inverted repeat was not detected downstream of the ORF.

The nucleotide sequences for *ori 44*, *ori 60*, and *ori 43* do not appear to exhibit significant homology with other published DNA sequences or with each other. The nucleotide sequences for these three *Bt* replication origins were used to survey the GenBank database for homologous prokaryotic sequences. In addition, homology comparisons were performed with published sequences for plasmid replication origins of the staphylococci and bacilli class I plasmids, pC194, pUB110, pRBH1, pE194, pC221, pT181, pUB112, pS194, pC223, and pSN2. The published sequences for pFTB14 from *Bacillus amyloliquifaciens* and the small cryptic plasmid pGI2 of *Bt ssp*. *thuringiensis* were also analyzed. In all cases, no significant homology was detected, either at the nucleotide or amino acid sequence level. Finally, no significant homologies were observed among the nucleotide and amino acid sequences derived for ori 44, ori 60, and ori 43, indicating that these three Bt replication origins are structurally unrelated to each other.

The conserved plus and minus origin sequences characteristic of the class I (single-stranded) DNA plasmids are also absent. This suggests that, in contrast to the small plasmids of *S.aureus* and *B.subtilis,* the native plasmids of *Bt* governed by *ori 44, ori 60*, or *ori 43* do not replicate via a single-strand DNA intermediate.

The prevalence of the *Bt* replication origins *ori 44*, *ori 60* and *ori 43* among *Bt* strains was also evaluated. Among fourteen different strains of *Bt* var. *kurstaki* that were studied, *ori 44* and *ori 43* were present in 70% of the strains; *ori 60* was present in 55% of the strains. The prevalence of these *Bt* replication origins was lower in *Bt* strains that were varieties other than *kurstaki*: *ori 44* was present in 38% of the forty-seven non-*kurstaki Bt* strains studied. The corresponding percentages for *ori 60* and *ori 43* were 23% and 26%, respectively.

### Example 4

### Construction of shuttle vector pEG597 containing ori 44

The *Bt* replication origin clone with *ori 44* contained on pEG588-8, described in Example 2 and shown in Figure 3A, was selected for use in the development of a shuttle vector exemplifying this invention. Plasmid pEG851 was derived from pEG588-8, as shown by the structural maps in Figure 3A, by inserting the 3.75 kb *Eco*RI-*Hind*III fragment of pEG588-8 into th*e E.coli* vector pTZ19u (cleaved at the *Eco*RI and *Hind*III sites), thereby replacing the multiple cloning site of pTZ19u with the cat gene and the *Bt* replication origin, *ori 44*.

The structural/restriction maps shown in Figure 11 illustrate the multistep strategy used to construct a shuttle vector based on *ori 44*, the *Bt* replication origin of the 44 mDa plasmid described in Example 3, using pEG851 as the starting point. An *Sph*I site located downstream of the *cat* gene was removed by digesting pEG851 with *Sph*I, using T4 polymerase and dNTPs to remove the 3' overhangs, and ligating the blunt ends together with T4 DNA ligase. Subsequently, the *Eco*RI site was replaced with a *Not*I site by cleaving the plasmid with *Eco*RI and inserting a *Not*I linker with EcoRI-compatible ends. Finally, a multiple cloning site, with the restriction endonuclease cleavage site sequence *Bam*HI, *Xba*I, *Pst*I, *Sst*I, *Sph*I, *Sma*I, *Hind*III, *Eco*RI, *Sal*I, *Not*I, was inserted at the unique HindIII site to yield a novel shuttle vector, designated as pEG597. A detailed description of the multiple cloning site construction is not necessary, since the use of synthetic oligonucleotides in molecular cloning and vector construction is well known to those skilled in the art. The sequence and orientation of the multiple cloning site in pEG597 was confirmed by DNA sequence analysis. To this end, template DNA was prepared from double-strand DNA using the procedure recommended in the Sequenase® sequencing kit manual.

### Example 5

### Construction of shuttle vector pEG853 containing ori 60 and shuttle vector pEG854 containing ori 43

In the development of the second and third shuttle vectors exemplified in this disclosure, the plasmid pEG588-14a (Table 1, Figure 3B) was used as the source for the Bt origin of replication, *ori 60*. The *Bt* replication origin *ori 60* was contained on a 2.3 kb cloned insert in pEG588-14a, which is shown in Figure 3, and this clone was used as the basis for constructing the shuttle vector now designated as pEG853.

The structural/restriction maps shown in Figure 12 illustrate the multistep procedure and strategy used to construct pEG853, and, thereafter, pEG854. The first shuttle vector, pEG597 described in Example 4, was used as the starting point in the development strategy. The *Bt* replication origin from the 60 mDa plasmid, *ori 60,* is contained on a 2.3 kb fragment flanked by *Sal*I sites in the plasmid pEG588-14a, shown in Figure 3B. A *Bam*HI site present in pEG588 was restored at one end of the cloned insert in pEG588-14a. This insert was ligated as a *Sal*I fragment to the 4.36 kb *Sal*I fragment of pEG597 containing *cat* and pTZ19u to yield pEG852, as shown in Figure 12. An *Sfi*I site was inserted at the *Xba*I site of pEG852 using an *Sfi*I:*Xba*I linker to restore the *Xba*I site on one side of the inserted linker, also as shown in Figure 12. The desired orientation of the linker shown in Figure 12 was selected by DNA sequence analysis. Subsequently, a multiple cloning site (MCS) was inserted at the unique *Bam*HI site of the unlabelled plasmid in Figure 12 to yield the shuttle vector pEG853, as shown in Figure 12. The multiple cloning site had the following restriction endonuclease cleavage site sequence: *Xba*I, *Pst*I, *Kpn*I, *Sma*I, *Avr*II, *Bam*HI, *Xho*I, *Sst*I, *Cla*I, *Hpa*I, *Sph*I, *Eag*I, *Sfi*I, *Sal*I*, Not*I. The orientation of the multiple cloning site was selected by restriction enzyme analysis and confirmed by DNA sequence analysis.

The second shuttle vector, pEG854, was developed using pEG853 as a starting point. The *Bt* origin of replication in pEG853, *ori 60*, was replaced with another *Bt* replication origin, *ori 43*, to yield shuttle vector pEG854. The replication origin from the 43 mDa plasmid, *ori 43*, contained on pEG588-13a (Table 1, Figure 3C) was localized to a 2.8 kb *Xba*I fragment within pEG588-13a by subcloning the 2.8 kb *Xba*I fragment into pEG588 to yield plasmid pEG599, as shown in Figure 3C. The subclone pEG599 was then shown to replicate in *Bt* by its ability to transform *Bt* strain HD73-26 to chloramphenicol resistance. To construct the shuttle vector pEG854, the *Bt* replication origin fragment containing *ori 60* in pEG853 was replaced by the *Bt* replication origin fragment containing *ori 43* in pEG599 by cleaving pEG853 with *Xba*I and inserting the 2.8 kb *Xba*I fragment from pEG599, all as shown in Figure 12. The resultant shuttle vector construct, pEG854, contains all of the restriction site modifications present in shuttle vector pEG853.

Both shuttle vectors, pEG853 and pEG854, are characterized by having a rare restriction endonuclease site, *Not*I, placed at each end of the pTZ19u sequence, to facilitate removal of this DNA segment. In addition, *Sfi*I and *Sal*I sites are located at each end of the DNA segment containing the *Bt* replication origin and multiple cloning site, to permit excision of non-*Bt* DNA sequences, i.e., the pTZ19u-*cat* DNA fragment. Furthermore, unique XbaI sites are placed at each end of the *Bt* replication origin DNA fragment in shuttle vectors pEG853 and pEG854 to permit the substitution of other replication origins, for instance, *ori 44* or other DNA fragments containing a *Bt* replication origin.

### Example 6

### Insertion of insecticidal Bt toxin genes into the Bt shuttle vectors

This Example demonstrates the utility of the shuttle vector plasmids as expression vectors for constructing recombinant *Bt* strains with specific toxin genes. To this end, two distinct *Bt* endotoxin genes were separately inserted into each of the three shuttle vectors, pEG597, pEG853 and pEG854 (see Figure 1). The first gene selected for this demonstration of overproduction in *Bt* was the *cryIIA* gene, previously known as *cryB1* (see Höfte and Whiteley, 1989). The *cryIIA* gene encodes the CryIIA protein (formerly referred to as P2 endotoxin), which exhibits insecticidal activity against both lepidopteran and dipteran insect larvae. The CryIIA protein accumulates as a small cuboidal crystal in *Bt* var. *kurstaki* strains but is typically absent in many other Bt varieties. The second selected gene, *cryIA(c)*, exhibits relatively potent insecticidal activity against a variety of lepidopteran insect pests. This gene was cloned from the transconjugant Bt strain HD73-26-10 that harbors a single cryIA(c) gene on a 44 mDa plasmid, which plasmid originated from *Bt* strain HD-263.

The *cryIIA* gene, located on a 4.0 kb *Bam*HI-*Hind*III fragment in pEG201, described by Donovan et al., *J.Biol.Chem.* (1988) *263*:561-567, was inserted into the shuttle vector pEG597 at the *Bam*HI and *Hind*III sites to generate pEG864. The same *cryIIA* gene from pEG201 was inserted as a 4.0 kb *Bam*HI-*Hpa*I fragment into the *Bam*HI and *Hpa*I sites of shuttle vectors pEG853 and pEG854 to yield plasmids pEG858 and pEG862, respectively. The second gene, *cryIA(c)*, was contained on a 5.0 kb *Sph*I-*Sal*I fragment isolated from a bacteriophage clone of 44 mDa plasmid DNA from *Bt* strain HD73-26-10, constructed by the inventor. This 5.0 kb DNA fragment was ligated into all three shuttle vectors, pEG597, pEG853, pEG854, at the *Sph*I and *Xho*I sites to yield pEG863, pEG857, and pEG861, respectively. The six constructs are listed in Table 2.

**Table 2**

| Shuttle vectors containing *Bt* endotoxin genes | | |
|---|---|---|
| Plasmid | Composition | Insert Orientation¹ |
| pEG863 | pEG597 + *cryIA(c)* | B |
| pEG864 | pEG597 + *cryIIA* | A |
| pEG857 | pEG853 + *cryIA(c)* | B |
| pEG858 | pEG853 + *cryIIA* | A |
| pEG861 | pEG854 + *cryIA(c)* | B |
| pEG862 | pEG854 + *cryIIA* | A |

| | | |
|---|---|---|
| ¹ Orientation of the cloned endotoxin gene with respect to the *cat* gene. | | |

These plasmids were introduced into the acrystalliferous *Bt* var. *kurstaki* strain HD73-26 by electroporation, described in detail below, and the transformants examined for toxin production.

Electroporation was performed according to the procedure described below. *Bt* strain HD73-26 was grown overnight at 30°C with vigorous shaking in 0.5X brain heart infusion and 0.5% glycerol (BHI medium). 500 µl of the overnight culture were suspended in 10 ml of BHI medium and grown for one hour at 30°C with shaking. The cells were pelleted in a preparative centrifuge at 4000 rpm using a JA20 rotor at 4°C for 10 minutes. The cell pellet was resuspended in 10 ml electroporation buffer (EB) containing 0.625 M sucrose and 1 mM MgCl₂ on ice and pelleted again. The resultant cell pellet was resuspended in 3.2 ml of EB and stored on ice until needed. Electroporation was performed using the BioRad Gene Pulser® apparatus. The electroporation cuvette contained 800 µl of *Bt* strain HD73-26 cells (in EB) and 10-20 µl plasmid DNA to be introduced into the recipient cells (see plasmids listed in Table 2), both suspended in sterile water. The Gene Pulser was set for 6250 V/cm, and 25 µF. After the contents of the cuvette were electrically pulsed, the cells were transferred to 1.6 ml of Luria broth (LB) containing 0.2 µg/ml chloramphenicol and incubated for two hours at 30°C with shaking. After this outgrowth period, the cells were plated on NSM plates (containing 23 g/l nutrient broth agar, 1 mM MgCl₂, 0.7 mM CaCl₂, 0.05 mM MnCl₂) containing 5 µg/ml chloramphenicol. The NSM plates were incubated overnight at 30°C.

Following completion of the electroporation procedure, the *Bt* strain HD73-26 chloramphenicol-resistant transformants, containing the toxin-encoding plasmids listed in Table 2, were characterized by restriction enzyme analysis and grown for 3 days at 30°C in liquid growth media, both in the presence of and absence of 5 µg/ml chloramphenicol. The *Bt* cultures were grown until fully sporulated and lysed, i.e., *Bt* spores and protein crystals that formed were released by cell lysis.

Protein crystals harvested from the lysed cultures of the *Bt* strain HD73-26 transformants were viewed by phase contrast microscopy and analyzed by SDS-polyacrylamide gel electrophoresis (SDS-PAGE). To prepare the toxin proteins for SDS-PAGE, aliquots (100 µl) of the sporulated *Bt* culture lysates were centrifuged in a microfuge for five minutes at room temperature. The spore-crystal pellets were washed once with 1 ml 10 mM Tris-HCl (pH 7.5), 1 mM EDTA, 1 mM EGTA, and dried in a Savant Speed-Vac Concentrator for 5-10 minutes. The dried pellets were suspended in SDS sample buffer at 95-100° C for 5-10 minutes. Insoluble material was removed by centrifugation in a microfuge for two minutes. Aliquots of the supernatant (10-20 µl) were loaded onto a 10% SDS-polyacrylamide gel for electrophoresis. Results of the SDS-PAGE analysis are shown in Figure 13.

*Bt* strain HD73-26 transformants harboring the *cryIIA* gene (on plasmids pEG864, pEG858, pEG862) produced large rounded or cuboidal crystals, often larger than the spore, that yielded an approximately 70 kDa protein on SDS-polyacrylamide gels, as shown on Figure 13. This protein comigrated with purified CryIIA (P2) crystal protein (used as a standard). Similarly, *Bt* transformants harboring the *cryIA(c)* gene (on plasmids pEG863, pEG857, pEG861) produced bipyramidal crystals typical of CryIA-type protein. This CryIA(c) protein comigrated with purified CryIA(c) crystal protein (used as a standard) on SDS-polyacrylamide gels at an apparent molecular weight of about 134 kDa. The presence or absence of chloramphenicol in the growth medium had no observable effect on toxin protein production.

These results demonstrate that the shuttle vectors are capable of, and useful for, expressing *Bt* toxin genes in *Bt* host strains. Cloned *Bt* toxin genes can be inserted into the shuttle vector of this invention, readily introduced in *Bt* by electroporation, and used to produce *Bt* toxin protein in recombinant *Bt* strains.

### Example 7

### Stability of toxin-encoding plasmids

The six shuttle vectors from Example 6, each containing *Bt* toxin genes as listed in Table 2, were tested for stability in *Bt* strain HD73-26 to determine whether they retained the stability of the parental vectors, i.e., the shuttle vectors without the inserted toxin gene. The transformants of *Bt* strain HD73-26 containing the toxin-encoding plasmids were grown for approximately 18 generations in the absence of selection (i.e., without chloramphenicol), plated onto LB plates, and single colonies transferred to NSM plates containing 5 µg/ml chloramphenicol. Chloramphenicol-resistant colonies were assumed to harbor the plasmid of interest.

Results of these studies are shown in Table 3. The data in Table 3 show the number of chloramphenicol-resistant colonies recovered from the total number of colonies tested after eighteen generations, e.g., 288/300. The fractional recovery rate is noted beneath the tabulated data. The plasmid designation is also indicated in parentheses, where applicable, to facilitate reference to Table 2.

**Table 3**

| Segregational stability of shuttle vectors | | | | | | |
|---|---|---|---|---|---|---|
| Shuttle vector (parent) | Replication origin | vector alone | +*cryIA(c)* | +*cryIIA* | +*cryIA(c)* -*E.coli* DNA | +*cryIIA* -*E.coli* DNA |
| pEG597 | *ori 44* | 288/300 | 145/200 | 137/200 | 135/200 | 159/200 |
| | | **0.96** (pEG597) | **0.72** (pEG863) | **0.68** (pEG864) | **0.68** | **0.80** |
| pEG853 | *ori 60* | 493/500 | 290/300 | 339/350 | 195/200 | 241/245 |
| | | **0.99** (pEG853) | **0.97** (pEG857) | **0.97** (pEG858) | **0.98** | **0.98** |
| pEG854 | *ori 43* | 400/400 | 198/200 | 195/200 | ND | ND |
| | | **1.0** (pEG854) | **0.99** (pEG861) | **0.98** (pEG862) | | |

Toxin-encoding plasmids derived from pEG853 and pEG854 (i.e., pEG857, pEG858, pEG861, pEG862) showed excellent stability in the absence of selection, whereas toxin-encoding plasmids derived from pEG597 (i.e., pEG863, pEG864) exhibited some instability (20-32% loss after 18 generations).

The toxin-encoding plasmids based on shuttle vectors pEG597 and pEG853 were also tested for stability after deletion of the DNA sequences derived from *E.coli* (pTZ19u) by *Not*I digestion and self-ligation of the vector fragment with T4 DNA ligase, and these data are shown in the last two columns of Table 3. These small toxin-encoding plasmids still contained the chloramphenicol acetyltransferase (*cat*) marker gene. As before, these plasmids were then evaluated for stability after introduction into *Bt* strain HD73-26 via electroporation. Removal of the pTZ19u sequences had little or no effect on the stability of the plasmids in the transformed *Bt* strain HD73-26, as evidenced by the data in Table 3.

The results of this Example indicate that small toxin-encoding plasmids comprised primarily of *Bt*-derived DNA can be maintained in recombinant *Bt* hosts without selection.

The recombinant plasmids harbored by *Bt* strain constructs obtained via use of the shuttle vector of this invention also exhibit low horizontal genetic transfer, i.e., via conjugal plasmid transfer, unlike native conjugative *Bt* toxin-encoding plasmids which presumably contain specific genes that mediate plasmid conjugal transfer. It is interesting to note that the 43 mDa cryptic plasmid and 44 mDa toxin gene plasmid, from which *ori 43* and *ori 44* were derived, are transferable via conjugation, but the 60 mDa toxin plasmid, the source of *ori 60*, is not.

### Example 8

### Introduction of a cloned toxin gene via the shuttle vector into a Bt strain containing toxin genes and bioassay of the recombinant Bt strain

To examine the effect of an introduced toxin gene on the insecticidal activity of a *Bt* strain harboring multiple toxin genes, the *cryIA(c)*-containing shuttle vector pEG863, listed in Table 2, was introduced into *Bt* var. *aizawai* EG6346, a strain lacking *cryIA*-type genes but possessing good insecticidal activity against *Spodoptera exigua* (beet armyworm). The *Bt* strain EG6346 transformant, designated EG6346/pEG863, was grown in liquid growth medium for three days at 30°C until the *Bt* culture was fully sporulated and lysed. The sporulated/lysed *Bt* culture contained primarily *Bt* spores and crystal protein and some cell debris; this mixture is referred to hereinafter as a spore/crystal preparation. The spore/crystal preparation of *Bt* strain EG6346/pEG863 was analyzed by SDS-PAGE and subsequently by Western blot analysis. For comparison, spore/crystal preparations of *Bt* strain EG6346 and a related strain, EG6345, were also examined. *Bt* strain EG6345 contains, in addition to the toxin genes of EG6346, a *cryIA(b)* gene. The photograph of the Coomassie-stained gel in Figure 14A shows three protein bands isolated from *Bt* strain EG6345 and two proteins bands each isolated from *Bt* strains EG6346 and EG6346/pEG863. The Western blot analysis shown in Figure 14B using CryIA(c) toxin-specific antibodies confirms that the CryIA(c) toxin is produced in the recombinant *Bt* strain EG6346/pEG863 but not in *Bt* strains EG6345 and EG6346.

The spore/crystal preparations of *Bt* strains EG6345, EG6346, and EG6346/pEG863 were used directly in quantitative bioassays against a variety of lepidopteran insect species. The insect species included *Heliothis zea* (corn earworm), *Spodoptera exigua* (beet armyworm), *Ostrinia nubilalis* (European cornborer), *Heliothis virescens* (tobacco budworm), *Trichoplusia ni* (cabbage looper), *Plutella xylostella* (diamondback moth). Activity against these lepidopteran insect larvae was determined by topically applying 100 µl of serially diluted spore/crystal preparations to 3 ml of an agar base artificial diet in a plastic feeding cup (600 mm² surface). One neonate larva was placed in each cup and scored for mortality after 7 days. LC₅₀ values were determined by probit analysis using an 8-dose testing procedure with 30 larvae per dose. The assays were performed in duplicate and the cumulative data analyzed.

Bioassay results are shown in Table 4 for each of *Bt* strain EG6345 (the native *Bt* strain), *Bt* strain EG6346 (the cured derivative of EG6345) and *Bt* strain EG6346/pEG863 (a recombinant *Bt* strain containing a toxin-encoding plasmid derived from the shuttle vector pEG597) against the six insect species tested. Displayed in Table 4 are the LC₅₀ concentrations of spore/crystal preparation, in nanoliters per cup (nl/cup) required to achieve 50% mortality of the insect larvae tested. The 95% confidence intervals are shown below the LC₅₀ values in the table.

**Table 4**

| Bioassay of *Bt* strains | | | | | | |
|---|---|---|---|---|---|---|
| Strain | LC₅₀ (nl/cup) | | | | | |
| | SE¹ | ON | HV | HZ | TN | PX |
| EG6345 | **123** | **26** | **>100** | **635** | **212** | **>100** |
| | 66-198 | 18-37 | | 522-789 | 100-374 | |
| EG6346 | **249** | **101** | **>100** | **894** | **367** | **>100** |
| | 200-311 | 79-130 | | 729-1132 | 134-1115 | |
| EG6346/pEG863 | **135** | **23** | **39** | **328** | **78** | **15** |
| | 110-165 | 13-47 | 28-62 | 267-416 | 63-95 | 6-23 |

| | | | | | | |
|---|---|---|---|---|---|---|
| ¹ SE = *Spodoptera exigua*, ON = *Ostrinia nubilalis*, *HZ* = *Heliothis zea*, HV = *Heliothis virescens*, TN = *Trichoplusia ni*, PX = *Plutella xylostella* | | | | | | |

The data in Table 4 were used to generate Figure 15, which is useful in comparing the relative insecticidal activities of these *Bt* strains.

The bioassay results in Table 4 and Figure 15 demonstrate that the recombinant *Bt* strain EG6346/pEG863 exhibits superior insecticidal activity compared to either of the two control strains, *Bt* strains EG6345 and EG6346.

These bioassay results demonstrate that the introduction of the *cryIA(c)*-containing pEG863 into the *Bt* strain EG6346 background enhances the insecticidal activity of the strain against all of the lepidopteran insects tested: *Spodoptera exigua*, *Ostrinia nubilalis, Heliothis virescens*, *Heliothis zea*, *Trichoplusia ni* and *Plutella xylostella.* Compared to *Bt* strain EG6345, the recombinant *Bt* strain EG6346/pEG863 also exhibits improved insecticidal activity against most of the insect species tested.

### Example 9

### Use of cotransformation for recombinant Bt strain development to introduce recombinant plasmids that are derived from Bt DNA and that lack selectable marker genes.

The plasmid shuttle vectors of this invention, e.g., pEG597, pEG853, and pEG854, are designed with pairs of unique restriction sites that may be used in the development of recombinant *Bt* strains, to construct recombinant plasmids containing only *Bt*-derived DNA and a synthetic multiple cloning site. Such recombinant plasmid constructs, optionally containing *Bt* toxin genes, are free of DNA derived from other biological sources and lack any antibiotic resistance genes.

Since these plasmid constructs lack a selectable marker gene, direct selection for *Bt* transformants containing these plasmids is not possible. However, these non-selectable plasmids may be introduced into *Bt* via cotransformation with another plasmid that does contain a selectable marker gene, such as one coding for antibiotic resistance. Electroporation studies are described below that demonstrate the feasibility of using cotransformation to introduce recombinant plasmids into *Bt*, using plasmids that are derived from *Bt* plasmid DNA and that lack antibiotic resistance genes.

To demonstrate that cotransformation can be used for this purpose, a *Bam*HI-*Sph*I restriction fragment containing a *cryIIIA-lacZ* gene fusion, constructed by the inventor, was inserted into the multiple cloning sites of plasmid shuttle vectors pEG853 and pEG854 to yield plasmids pEG871 and pEG872, respectively. Linear restriction maps of plasmids pEG871 and pEG872 are shown in Figure 16.

The *cryIIIA-lacZ* gene fusion serves as a convenient marker gene for cotransformation studies. The *cryIIIA-lacZ* gene encodes a fusion protein with beta-galactosidase activity. This enzymatic activity may be used to convert the chromogenic dye indicator X-Gal (5-bromo-4-chloro-3-indolyl β-D-galactopyranoside) to a blue pigment. *Bt* colonies expressing the *cryIIIA-lacZ* gene protein product are blue in color when grown on plates containing X-Gal. Thus, a recombinant plasmid containing the *cryIIIA-lacZ* gene fusion but lacking a selectable antibiotic resistance gene may still be detected in a *Bt* transformant.

The shuttle vector pEG147, shown in Figure 17, was chosen to serve as the selectable plasmid in the cotransformation experiment. This vector contains the tetracycline resistance gene (*tet*) and the replication origin of pBC16, which is compatible with *ori 43*, *ori 44,* an*d ori 60*. Plasmid pEG147 was constructed by inserting an *Eco*RI fragment from pBC16, containing *tet* and the pBC16 replication origin, into the *Ssp*I site of *E.coli* cloning vector pUC18.

In preparation for cotransformation, 5-10 micrograms (µg) of the *cryIIIA-lacZ* plasmids pEG871 and pEG872 were cleaved with the restriction endonucleases *Sfi*I and *Not*I and the resultant DNA fragments ligated with T4 DNA ligase. Digestion with *Sfi*I releases a DNA fragment from the plasmids that contains the *Bt* replication origin and the *cryIIIA-lacZ* gene fusion. Self-ligation of this *Sfi*I fragment generates a recombinant plasmid containing a *Bt* replication origin and the *cryIIIA-lacZ* gene fusion inserted in the multiple cloning site, as shown diagramatically in Figure 18. Simultaneous digestion of pEG871 and pEG872 with *Not*I reduces the likelihood that the DNA fragments containing the cat marker gene or the *E.coli-*derived pTZ19u will ligate to the *Sfi*I fragments containing the *Bt* replication origin and *cryIIIA-lacZ* gene.

For cotransformation, the products of both ligation reactions (i.e., the ligations of the pEG871 and pEG872 restriction digests) were separately combined with 0.1 µg each of pEG147 (containing the tetracycline resistance gene), and the DNA mix was used to transform *Bt* strain HD73-26 to tetracycline resistance via electroporation. Electroporation was performed as described previously in Example 6, with the exception that chloramphenicol was not added to the cells prior to the outgrowth period. After the outgrowth period, the cells were spread with 50 µl 2% X-Gal onto NSM plates containing 10 µg/ml tetracycline. Plates were incubated overnight at 30°C.

Among the tetracycline-resistant colonies recovered from the electroporation procedure, about 2-5% were blue and chloramphenicol-sensitive. This indicated that these tetracycline-resistant colonies contained, in addition to pEG147 with its *tet* gene, the *cryIIIA-lacZ* gene but lacked the chloramphenicol resistance gene (*cat*) and presumably lacked the pTZ19u segment (with its *E.coli-*functional origin of replication) from pEG871 and pEG872. The recovery of blue, chloramphenicol-sensitive colonies demonstrates that the self-ligated *Sfi*I fragments from pEG871 and pEG872 were successfully introduced into *Bt* by cotransformation, resulting in recombinant plasmids that contained the *cryIIIA-lacZ* gene but lacked antibiotic resistance genes and an *E.coli*-functional origin of replication, as shown diagramatically in Figure 18.

Removal of pEG147, containing the tetracycline resistance gene, may be achieved by subsequently selecting for tetracycline-sensitive colonies. The resultant tetracycline-sensitive *Bt* strain would contain only a recombinant plasmid bearing a *Bt* replication origin and the *cryIIIA-lacZ* gene inserted into the multiple cloning site, both derived from the plasmid shuttle vectors pEG871 or pEG872.

The same procedure may be adapted to introduce cloned toxin genes in *Bt* on recombinant plasmids derived from *Bt* DNA. *Bt* colonies that contain the toxin-encoding recombinant plasmid derived from the shuttle vector may be identified among the tetracycline-resistant colonies by using conventional colony blot hybridization procedures and radiolabelled toxin gene-specific hybridization probes, instead of a *lacZ* marker gene.

In such a procedure, the tetracycline-resistant colonies are partially transferred to a nitrocellulose filter where they are lysed, and the released plasmid DNA is immobilized on the filter. Filters containing the immobilized plasmid DNAs are incubated with a radiolabelled hybridization probe consisting either of the cloned toxin gene or the appropriate *Bt* replication origin fragment. Hybridization of the immobilized plasmid DNA to either of these probes can be detected by autoradiography, thereby enabling identification of colonies containing the toxin-encoding plasmid. Tetracycline-resistant clones containing the recombinant toxin-encoding plasmid may be subsequently tested for growth on chloramphenicol to demonstrate absence of the chloramphenicol resistance gene, *cat.* Removal of the selectable plasmid pEG147, containing the tetracycline resistance gene, may be achieved by subsequently selecting for tetracycline-sensitive *Bt* colonies. The resultant tetracycline-sensitive *Bt* strain would contain only a recombinant plasmid bearing a *Bt* replication origin and a *Bt* toxin gene inserted into the multiple cloning site.

There are no known reports in the prior art that describe cotransformation of *Bt*. The development of a highly efficient electroporation procedure for *E.coli* (10¹⁰ transformants/µg DNA) has led other researchers to believe that a high level of cotransformation could be achieved in *E.coli* with this procedure; see Dower et al., "High efficiency transformation of *E.coli* by high voltage electroporation", *Nucleic Acids Res*. 1988, *16*:6127-6145. In contrast, published reports of *Bt* transformation (not cotransformation) using known electroporation protocols have indicated relatively low transformation efficiencies, about 10⁵-10⁷ transformants/µg DNA, with only a small percentage of the *Bt* cells actually being transformed. Because of the reported inefficiency in the transformation of *Bt*, the inventor did not expect good cotransformation efficiencies to be realized in his cotransformation studies with *Bt*. Nevertheless, using 1 µg each of pEG871 and pEG147 to transform *Bt* strain HD73-26 to tetracycline resistance, 12% of the tetracycline-resistant colonies were found to harbor pEG871, as evidenced by the formation of blue colonies on plates containing X-Gal. When 0.1 µg of pEG147 and 1 µg of pEG871 were used to transform *Bt* strain HD73-26, a cotransformation frequency of 25% was achieved. In other words, 25% of the tetracycline-resistant colonies containing pEG147 also contained pEG871. These results demonstrate that a high level of cotransformation can in fact be achieved with *Bt* and that the plasmid shuttle vectors of this invention may be utilized to develop novel recombinant *Bt* strains that are free of foreign DNA from non-*Bt* biological sources. The use of cotransformation in recombinant *Bt* strain development thus facilitates the introduction into *Bt* of recombinant toxin-encoding plasmids which contain only *Bt*-derived DNA and which lack antibiotic resistance genes.

To assure the availability of materials to those interested members of the public upon issuance of a patent on the present application, deposits of the following microorganisms were made prior to the filing of the present application with the ARS Patent Collection, Agricultural Research Culture Collection, Northern Regional Research Laboratory (NRRL), 1815 North University Street, Peoria, Illinois 61064, as indicated in the following Table 5:

**Table 5**

| Bacterial Strain | Corresponding Plasmid | NRRL Accession | No. Date of Deposit |
|---|---|---|---|
| B.thuringiensis HD73-26 | | B-18508 | June 12, 1989 |
| B.thuringiensis EG6345 | | B-18633 | March 27, 1990 |
| E.coli EG1597 | pEG597 | B-18630 | March 27, 1990 |
| E.coli EG7529 | pEG853 | B-18631 | March 27, 1990 |
| E.coli EG7534 | pEG854 | B-18632 | March 27, 1990 |

These microorganism deposits were made under the provisions of the "Budapest Treaty on the International Recognition of the Deposit of Microorganisms for the Purposes of Patent Procedure".

Bt strains HD-73 and HD-263 (progenitors of HD73-26 and HD263-6, respectively) are available from USDA, ARS, Cotton Insects Research Unit, P.O. Box 1033, Brownsville, Texas 78520.

The present invention may be embodied in other specific forms without departing from the essential attributes thereof and, accordingly, reference should be made to the appended claims, rather than to the foregoing specification as indicating the scope of the invention.

## Claims

1. A plasmid shuttle vector, useful for introducing genes into a *Bacillus thuringiensis* (*Bt*) host, characterized in that the vector is a plasmid capable of transforming a *Bt* bacterium and contains (i) a removable DNA restriction fragment containing an origin of replication functional in *E. coli;* (ii) a multiple cloning site, with multiple restriction endonuclease cleavage sites to facilitate cloning of an inserted *Bt* gene; and (iii) a removable origin of replication derived from a *Bt* plasmid and being selected from the group consisting of *ori 44*, *ori 60* and *ori 43*, wherein *ori 44, ori 60*, and *ori 43* have the respective nucleotide sequences shown in Figures 5, 6 and 7, the *Bt-*derived origin of replication being adjacent to the multiple cloning site, and the *Bt*-derived origin of replication and the multiple cloning site having flanking restriction endonuclease cleavage sites that enable the *Bt*-derived origin of replication and the multiple cloning site to be isolated from the plasmid shuttle vector as a single combined DNA fragment and to be introduced, after self-ligation, as a functional plasmid into a Bt host.

2. The plasmid shuttle vector of claim 1 further characterized in that the flanking restriction endonuclease cleavage sites are identical, such sites being unique to the plasmid shuttle vector and being absent from a gene of interest to be introduced into a *Bt* host via the shuttle vector.

3. The plasmid shuttle vector of claim 1 or 2 further characterized in that the restriction endonuclease cleavage sites are selected from the group of *Sal*I and *Sfi*I sites.

4. The plasmid shuttle vector of claim 1 or 2 further characterized in that the vector contains two pairs of flanking restriction endonuclease cleavage sites, one pair of flanking restriction sites being *Sal*I sites and the other pair of flanking restriction sites being *Sfi*I sites.

5. The plasmid shuttle vector of any one of claims 1 to 4 further characterized in that the plasmid shuttle vector has two identical restriction endonuclease cleavage sites that flank the removable DNA restriction fragment containing the origin of replication functional in *E. coli*, such sites being unique to the plasmid shuttle vector and being absent from a gene of interest to be introduced into a *Bt* host via the shuttle vector.

6. The plasmid shuttle vector of claim 5 further characterized in that the two identical restriction endonuclease cleavage sites flanking the *E. coli*-functional origin of replication are *Not*I sites.

7. The plasmid shuttle vector of any one of claims 1 to 6 further characterized in that the plasmid shuttle vector has two identical restriction endonuclease cleavage sites that flank the removable *Bt*-derived origin of replication, such sites being unique to the plasmid shuttle vector.

8. The plasmid shuttle vector of claim 7 further characterized in that the two identical restriction endonuclease cleavage sites flanking the removable *Bt*-derived origin of replication are *Xba*I sites.

9. The plasmid shuttle vector of any one of claims 1 to 8 further characterized in that the plasmid shuttle vector has at least one selectable marker gene conferring antibiotic resistance, the marker gene being present on a removable DNA restriction fragment in the plasmid.

10. The plasmid shuttle vector of claim 9 further characterized in that the selectable marker gene conferring antibiotic resistance is functional in *Bt*.

11. The plasmid shuttle vector of claim 10 further characterized in that the selectable marker gene is functional in *Bt* and is located adjacent to the *Bt*-derived origin of replication.

12. The plasmid shuttle vector of claim 10 further characterized in that the selectable marker gene is selected from the group consisting of a chloramphenicol resistance gene, a tetracycline resistance gene, and a kanamycin resistance gene.

13. The plasmid shuttle vector of claim 9 further characterized in that the selectable marker gene conferring antibiotic resistance is functional in *E. coli*.

14. The plasmid shuttle vector of claim 13 further characterized in that the selectable marker gene is functional in *E. coli* and is located adjacent to or within the DNA fragment containing the origin of replication functional in *E. coli*.

15. The plasmid shuttle vector of claim 13 further characterized in that the selectable marker gene is selected from the group consisting of an ampicillin resistance gene, a chloramphenicol resistance gene, a tetracycline resistance gene, and a kanamycin resistance gene.

16. The plasmid shuttle vector of any one of claims 1 to 15 further characterized in that the multiple cloning site has restriction endonuclease cleavage sites in the following sequence, *Xba*I *Pst*I *Kpn*I *Sma*I *Avr*II *Bam*HI *Xho*I *Sst*I *Cla*I *Hpa*I *Sph*I *Eag*I *Sfi*I *Sal*I *Not*I, and the *Xba*I site is located adjacent to the *Bt*-derived origin of replication.

17. The plasmid shuttle vector of claim 16 further characterized in that the vector contains *ori 60* as the *Bt*-derived origin of replication and is plasmid pEG853 harbored in *E. coli* strain EG7529, deposited with the Agricultural Research Culture Collection (NRRL) and assigned NRRL Accession No. B-18631.

18. The plasmid shuttle vector of claim 16 further characterized in that the vector contains *ori 43* as the *Bt*-derived origin of replication and is plasmid pEG854 harbored in *E. coli* strain EG7534, deposited with the Agricultural Research Culture Collection (NRRL) and assigned NRRL Accession No. B-18632.

19. The plasmid shuttle vector of any one of claims 1 to 15 further characterized in that the multiple cloning site has restriction endonuclease cleavage sites in the following sequence, *Bam*HI *Xho*I *Pst*I *Sst*I *Sph*I *Sma*I *Hind*III *Eco*RI *Sal*I *Not*I, and the *Bam*HI site is located adjacent to the *Bt*-derived origin of replication.

20. The plasmid shuttle vector of claim 19 further characterized in that the vector contains *ori 44* as the *Bt-*derived origin of replication and is plasmid pEG597 harbored in *E. coli* strain EG1597, deposited with the Agricultural Research Culture Collection (NRRL) and assigned NRRL Accession No. B-18630.

21. A recombinant *Bt* strain characterized by having a toxin-encoding, functional recombinant plasmid derived from the plasmid shuttle vector of any one of claims 1 to 20.

22. The recombinant Bt strain of claim 21 further characterized in that the recombinant Bt strain and its functional recombinant plasmid are essentially free of DNA from a non-Bt biological source.

23. The recombinant *Bt* strain of claim 21 or 22 further characterized in that the functional recombinant plasmid consists essentially of a *Bt*-derived origin of replication and at least one *Bt* toxin gene inserted into a multiple cloning site.

24. An isolated, purified DNA fragment characterized by being designated as *ori 44*, useful as an origin of replication functional in *Bt*, coding for the amino acid sequence shown in Figure 5.

25. An isolated, purified DNA fragment according to claim 24 further characterized in that the coding region extends from nucleotide bases 797 to 1732 in the nucleotide base sequence shown in Figure 5.

26. An isolated, purified DNA fragment characterized by being designated a*s ori 60*, useful as in origin of replication functional in *Bt*, coding for the amino acid sequence shown in Figure 6.

27. An isolated, purified DNA fragment according to claim 26 wherein the coding region extends from nucleotide bases 627 to 1844 in the nucleotide base sequence shown in Figure 6.

28. An isolated, purified DNA fragment characterized by being designated as *ori 43*, useful as an origin of replication functional in *Bt*, coding for the amino acid sequence shown in Figure 7.

29. An isolated, purified DNA fragment according to claim 28 further characterized in that the coding region extends from nucleotide bases 829 to 2358 in the nucleotide base sequence shown in Figure 7.

30. A recombinant plasmid characterized in that it contains the DNA fragment of any one of claims 24 to 29.

31. A biologically pure culture of a *Bacillus thuringiensis* bacterium characterized in that it is transformed with the recombinant plasmid of claim 30.

32. A process for the preparation of a DNA fragment according to any one of Claims 24 to 29 which comprises isolating said fragment from a vector containing it by using suitable restriction enzymes and purifying it by using appropriate methods.

## Patentansprüche

1. Plasmid-Shuttlevektor, verwendbar zur Einführung von Genen in einen *Bacillus thuringiensis*-Wirt (*Bt*) und dadurch gekennzeichnet, daß der Vektor ein Plasmid mit der Fähigkeit zur Transformation eines *Bt-*Bakteriums ist und enthält (i) ein entfernbares DNA-Restriktionsfragment, enthaltend einen in *E.coli* funktionellen Replikationsstartpunkt; (ii) eine Mehrfachclonierungsstelle mit mehreren Restriktionsendonuclease-Spaltstellen zur Erleichterung der Clonierung eines eingesetzten *Bt*-Gens; und (iii) einen entfernbaren, aus einem *Bt*-Plasmid stammenden Replikationsstartpunkt, ausgewählt aus *ori 44*, *ori 60* und *ori 43*, wobei *ori 44*, *ori 60* und *ori 43* die in Figur 5, 6 bzw. 7 gezeigte Nucleotidsequenz aufweisen, der aus *Bt* stammende Replikationsstartpunkt der Mehrfachclonierungstelle benachbart ist und der aus *B*t stammende Replikationsstartpunkt und die Mehrfachclonierungsstelle flankierende Restriktionsendonuclease-Spaltstellen besitzen, welche die Isolierung des aus *Bt* stammenden Replikationsstartpunktes und der Mehrfachclonierungsstelle aus dem Plasmid-Shuttlevektor als ein einzelnes verbundenes DNA-Fragment ermöglichen sowie nach Selbstligierung die Einführung als ein funktionelles Plasmid in einen *Bt*-Wirt.

2. Plasmid-Shuttlevektor nach Anspruch 1, ferner dadurch gekennzeichnet, daß die flankierenden Restriktionsendonuclease-Spaltstellen identisch sind, wobei derartige Stellen nur einmal im Plasmid-Shuttlevektor vorhanden sind und in einem interessierenden Gen fehlen, das über den Shuttle-Vektor in einen *Bt*-Wirt eingeführt werden soll.

3. Plasmid-Shuttlevektor nach Anspruch 1 oder 2, ferner dadurch gekennzeichnet, daß die Restriktionsendonuclease-Spaltstellen aus *Sal*I- und *Sfi*I-Stellen ausgewählt werden.

4. Plasmid-Shuttlevektor nach Anspruch 1 oder 2, ferner dadurch gekennzeichnet, daß der Vektor zwei Paare flankierender Restriktionsendonuclease-Spaltstellen enthält, wobei ein Paar flankierender Restriktions-Spaltstellen *Sal*I-Stellen und das andere Paar flankierender Restriktions-Spaltstellen *Sfi*I-Stellen sind.

5. Plasmid-Shuttlevektor nach einem der Ansprüche 1 bis 4, ferner dadurch gekennzeichnet, daß der Plasmid-Shuttlevektor zwei identische Restriktionsendonuclease-Spaltstellen besitzt, die das entfernbare DNA-Restriktionsfragment flankieren, enthaltend den in *E.coli* funktionellen Replikationsstartpunkt, wobei derartige Stellen nur einmal im Plasmid-Shuttlevektor vorhanden sind und in einem interessierenden Gen fehlen, das über den Shuttle-Vektor in einen *Bt*-Wirt eingeführt werden soll.

6. Plasmid-Shuttlevektor nach Anspruch 5, ferner dadurch gekennzeichnet, daß die zwei identischen Restriktionsendonuclease-Spaltstellen, die den in *E.coli* funktionellen Replikationsstartpunkt flankieren, *Not*I-Stellen sind.

7. Plasmid-Shuttlevektor nach einem der Ansprüche 1 bis 6, ferner dadurch gekennzeichnet, daß der Plasmid-Shuttlevektor zwei identische Restriktionsendonuclease-Spaltstellen aufweist, die den aus *Bt* stammenden, entfernbaren Replikationsstartpunkt flankieren, wobei derartige Stellen nur einmal im Plasmid-Shuttlevektor vorhanden sind.

8. Plasmid-Shuttlevektor nach Anspruch 7, ferner dadurch gekennzeichnet, daß die zwei identischen Restriktionsendonuclease-Spaltstellen, die den aus *Bt* stammenden, entfernbaren Replikationsstartpunkt flankieren, *Xba*I-Stellen sind.

9. Plasmid-Shuttlevektor nach einem der Ansprüche 1 bis 8, ferner dadurch gekennzeichnet, daß der Plasmid-Shuttlevektor mindestens ein Antibiotikaresistenz verleihendes selektierbares Markergen aufweist, wobei das Markergen im Plasmid auf einem entfernbaren DNA-Restriktionsfragment vorhanden ist.

10. Plasmid-Shuttlevektor nach Anspruch 9, ferner dadurch gekennzeichnet, daß das Antibiotikaresistenz verleihende, selektierbare Markergen in *Bt* funktionell ist.

11. Plasmid-Shuttlevektor nach Anspruch 10, ferner dadurch gekennzeichnet, daß das selektierbare Markergen in *Bt* funktionell ist und dem aus *Bt* stammenden Replikationsstartpunkt benachbart liegt.

12. Plasmid-Shuttlevektor nach Anspruch 10, ferner dadurch gekennzeichnet, daß das selektierbare Markergen ausgewählt wird aus einem Chloramphenicol-Resistenzgen, einem Tetracyclin-Resistenzgen und einem Kanamycin-Resistenzgen.

13. Plasmid-Shuttlevektor nach Anspruch 9, ferner dadurch gekennzeichnet, daß das Antibiotikaresistenz verleihende, selektierbare Markergen in *E.coli* funktionell ist.

14. Plasmid-Shuttlevektor nach Anspruch 13, ferner dadurch gekennzeichnet, daß das selektierbare Markergen in *E.coli* funktionell ist und neben oder innerhalb des DNA-Fragmentes liegt, welches den in *E.coli* funktionellen Replikationsstartpunkt enthält.

15. Plasmid-Shuttlevektor nach Anspruch 13, ferner dadurch gekennzeichnet, daß das selektierbare Markergen ausgewählt wird aus einem Ampicillin-Resistenzgen, einem Chloramphenicol-Resistenzgen, einem Tetracyclin-Resistenzgen und einem Kanamycin-Resistenzgen.

16. Plasmid-Shuttlevektor nach einem der Ansprüche 1 bis 15, ferner dadurch gekennzeichnet, daß die Mehrfachclonierungsstelle Restriktionsendonuclease-Spaltstellen in der folgenden Reihenfolge aufweist *Xba*I *Pst*I *Kpn*I *Sma*I *Avr*II *Bam*HI *Xho*I *Sst*I *Cla*I *Hpa*I *Sph*I *Eag*I *Sfi*I *Sal*I *Not*I, wobei die *Xba*I-Stelle dem aus *Bt* stammenden Replikationsstartpunkt benachbart liegt.

17. Plasmid-Shuttlevektor nach Anspruch 16, ferner dadurch gekennzeichnet, daß der Vektor *ori 60* als den aus *Bt* stammenden Replikationsstartpunkt enthält und Plasmid pEG853 ist, enthalten im *E.coli*-Stamm EG7529, der bei der "Agricultural Research Culture Collection" (NRRL) hinterlegt und dem die NRRL-Hinterlegungsnr. B-18631 zugewiesen wurde.

18. Plasmid-Shuttlevektor nach Anspruch 16, ferner dadurch gekennzeichnet, daß der Vektor *ori 43* als den aus *Bt* stammenden Replikationsstartpunkt enthält und Plasmid pEG854 ist, enthalten im *E.coli*-Stamm EG7534, der bei der "Agricultural Research Culture Collection" (NRRL) hinterlegt und dem die NRRL-Hinterlegungsnr. B-18632 zugewiesen wurde.

19. Plasmid-Shuttlevektor nach einem der Ansprüche 1 bis 15, ferner dadurch gekennzeichnet, daß die Mehrfachclonierungsstelle Restriktionsendonuclease-Spaltstellen in der folgenden Reihenfolge aufweist *Bam*HI *Xho*l *Pst*I *Sst*I *Sph*I *Sma*I *Hind*III *Eco*RI *Sal*I *Not*I, wobei die *Bam*HI-Stelle dem aus *Bt* stammenden Replikationsstartpunkt benachbart liegt.

20. Plasmid-Shuttlevektor nach Anspruch 19, ferner dadurch gekennzeichnet, daß der Vektor *ori 44* als den aus *Bt* stammenden Replikationsstartpunkt enthält und Plasmid pEG597 ist, enthalten im *E.coli*-Stamm EG1597, der bei der "Agricultural Research Culture Collection" (NRRL) hinterlegt wurde und dem die NRRL-Hinterlegungsnr. B-18630 zugewiesen wurde.

21. Rekombinanter *Bt*-Stamm, gekennzeichnet durch den Besitz eines Toxin codierenden, funktionellen rekombinanten Plasmids, das aus dem Plasmid-Shuttlevektor nach einem der Ansprüche 1 bis 20 erhalten wurde.

22. Rekombinanter *Bt*-Stamm nach Anspruch 21, ferner dadurch gekennzeichnet, daß der rekombinante *Bt*-Stamm und dessen funktionelles rekombinantes Plasmid im wesentlichen frei sind von DNA aus biologischen nicht-*Bt*-Ausgangsmaterialien.

23. Rekombinanter *Bt*-Stamm nach Anspruch 21 oder 22, ferner dadurch gekennzeichnet, daß das funktionelle rekombinante Plasmid im wesentlichen aus einem aus *Bt* stammenden Replikationsstartpunkt und mindestens einem *Bt*-Toxingen besteht, eingesetzt in eine Mehrfachclonierungsstelle.

24. Isoliertes, gereinigtes DNA-Fragment, gekennzeichnet durch die Bezeichnung als *ori 44*, dadurch daß es verwendbar ist als ein in *Bt* funktioneller Replikationsstartpunkt und dadurch, daß es die in Figur 5 gezeigte Aminosäuresequenz codiert.

25. Isoliertes, gereinigtes DNA-Fragment nach Anspruch 24, ferner dadurch gekennzeichnet, daß sich der codierende Bereich in der in Figur 5 gezeigten Nucleotidbasensequenz von Nucleotidbase 797 bis 1732 erstreckt.

26. Isoliertes, gereinigtes DNA-Fragment, gekennzeichnet durch die Bezeichnung als *ori 60*, dadurch daß es verwendbar ist als ein in *Bt* funktioneller Replikationsstartpunkt und dadurch, daß es die in Figur 6 gezeigte Aminosäuresequenz codiert.

27. Isoliertes, gereinigtes DNA-Fragment nach Anspruch 26, wobei sich der codierende Bereich in der in Figur 6 gezeigten Nucleotidbasensequenz von Nucleotidbase 627 bis 1844 erstreckt.

28. Isoliertes, gereinigtes DNA-Fragment, gekennzeichnet durch die Bezeichnung als *ori 43*, dadurch daß es verwendbar ist als ein in *Bt* funktioneller Replikationsstartpunkt und dadurch, daß es die in Figur 7 gezeigte Aminosäuresequenz codiert.

29. Isoliertes, gereinigtes DNA-Fragment nach Anspruch 28, ferner dadurch gekennzeichnet, daß sich der codierende Bereich in der in Figur 7 gezeigten Nucleotidbasensequenz von Nucleotidbase 829 bis 2358 erstreckt.

30. Rekombinantes Plasmid, dadurch gekennzeichnet, daß es das DNA-Fragment nach einem der Ansprüche 24 bis 29 enthält.

31. Biologisch reine Kultur eines *Bacillus thuringiensis*-Bakterium, dadurch gekennzeichnet, daß es mit dem rekombinanten Plasmid nach Anspruch 30 transformiert ist.

32. Verfahren zur Herstellung eines DNA-Fragments nach einem der Ansprüche 24 bis 29, umfassend die Isolierung dieses Fragments mit einem geeigneten Restriktionsenzym aus einem Vektor, der es enthält, und die Reinigung des Fragments mit einem geeigneten Verfahren.

## Revendications

1. Vecteur navette de plasmide, utilisable pour introduire des gènes dans *Bacillus thuringiensis* (*Bt*) comme hôte, caractérisé en ce que le vecteur est un plasmide capable de transformer une bactérie *Bt* et contient (i) un fragment de restriction d'ADN éliminable contenant une origine de réplication fonctionnelle dans *E. coli*; (ii) un site de clonage multiple, avec des sites de coupure par les endonucléases de restriction multiples pour faciliter le clonage d'un gène *Bt* inséré; et (iii) une origine de réplication éliminable dérivant d'un plasmide *Bt* et choisie parmi *ori 44*, *ori 60* et *ori 43*, où *ori 44*, *ori 60*, et *ori 43* ont les séquences de nucléotide respectives indiquées dans les figures 5, 6 et 7, l'origine de réplication dérivée de *Bt* étant adjacente au site de clonage multiple et l'origine de réplication dérivée de *Bt* et le site de clonage multiple ayant des sites de coupure par les endonucléases de restriction adjacents qui permettent à l'origine de réplication dérivée de *Bt* et au site de clonage multiple d'être isolés du vecteur navette de plasmide sous la forme d'un fragment d'ADN combiné unique et d'être introduits, après auto-ligature, en tant que plasmide fonctionnel dans un hôte *Bt*.

2. Vecteur navette de plasmide selon la revendication 1, caractérisé en outre en ce que les sites de coupure par les endonucléases de restriction adjacents sont identiques, ces sites étant uniques pour le vecteur navette de plasmide et étant absents d'un gène intéressant à introduire dans un hôte *Bt* par l'intermédiaire du vecteur navette.

3. Vecteur navette de plasmide selon les revendications 1 ou 2, caractérisé en outre en ce que les sites de coupure par les endonucléases de restriction sont choisis parmi les sites *Sal*I et *Sfi*I.

4. Vecteur navette de plasmide selon les revendications 1 ou 2, caractérisé en outre en ce que le vecteur contient deux paires de sites de coupure par les endonucléases de restriction adjacents, une paire de sites de restriction adjacents étant des sites *Sal*I et l'autre paire de sites de restriction adjacents étant des sites *Sfi*I.

5. Vecteur navette de plasmide selon l'une quelconque des revendications 1 à 4, caractérisé en outre en ce que le vecteur navette de plasmide a deux sites de coupure par les endonucléases de restriction identiques qui sont adjacents au fragment de restriction de l'ADN éliminable contenant l'origine de réplication fonctionnelle dans *E. coli*, ces sites étant uniques dans le vecteur navette de plasmide et étant absents d'un gène intéressant à introduire dans un hôte *Bt* par l'intermédiaire du vecteur navette.

6. Vecteur navette de plasmide selon la revendication 5, caractérisé en outre en ce que les deux sites de coupure par les endonucléases de restriction identiques adjacents à l'origine de réplication fonctionnelle de *E. coli* sont des sites *Not*I.

7. Vecteur navette de plasmide selon l'une quelconque des revendications 1 à 6, caractérisé en outre en ce que le vecteur navette de plasmide a deux sites de coupure par les endonucléases de restriction identiques qui sont adjacents à l'origine de réplication dérivée de *Bt* éliminable, ces sites étant uniques dans le vecteur navette de plasmide.

8. Vecteur navette de plasmide selon la revendication 7, caractérisé en outre en ce que les deux sites de coupure par les endonucléases de restriction identiques adjacents à l'origine de réplication dérivée de *Bt* éliminable sont des sites *Xba*I.

9. Vecteur navette de plasmide selon l'une quelconque des revendications 1 à 8, caractérisé en outre en ce que le vecteur navette de plasmide a au moins un gène marqueur pouvant être choisi conférant la résistance aux antibiotiques, le gène marqueur étant présent sur un fragment de restriction d'ADN éliminable dans le plasmide.

10. Vecteur navette de plasmide selon la revendication 9, caractérisé en outre en ce que le gène marqueur sélectionnable conférant la résistance aux antibiotiques est fonctionnel dans *Bt*.

11. Vecteur navette de plasmide selon la revendication 10, caractérisé en outre en ce que le gène marqueur sélectionnable est fonctionnel dans *Bt* et est adjacent à l'origine de réplication dérivée de *Bt*.

12. Vecteur navette de plasmide selon la revendication 10, caractérisé en outre en ce que le gène de marqueur sélectionnable est choisi parmi un gène de résistance au chloramphénicol, un gène de résistance à la tétracycline, et un gène de résistance à la kanamycine.

13. Vecteur navette de plasmide selon la revendication 9, caractérisé en outre en ce que le gène de marqueur sélectionnable conférant la résistance aux antibiotiques est fonctionnel dans *E. coli*.

14. Vecteur navette de plasmide selon la revendication 13, caractérisé en outre en ce que le gène de marqueur sélectionnable est fonctionnel dans *E. coli* et est adjacent au fragment d'ADN contenant l'origine de réplication fonctionnelle dans *E. coli*, ou est à l'intérieur de celui-ci.

15. Vecteur navette de plasmide selon la revendication 13, caractérisé en outre en ce que le gène de marqueur sélectionnable est choisi parmi un gène de résistance à l'ampicilline, un gène de résistance au chloramphénicol, un gène de résistance à la tétracycline, et un gène de résistance à la kanamycine.

16. Vecteur navette de plasmide selon l'une quelconque des revendications 1 à 15, caractérisé en outre en ce que le site de clonage multiple a des sites de coupure par les endonucléases de restriction dans l'ordre suivant, *Xba*I *Pst*I *Kpn*I *Sma*I *Avr*II *Bam*HI *Xho*I *Sst*I *Cla*I *Hpa*I *Sph*I *Eag*I *Sfi*I *Sal*I *Not*I, et le site *Xba*I est adjacent à l'origine de réplication dérivée de *Bt*.

17. Vecteur navette de plasmide selon la revendication 16, caractérisé en outre en ce que le vecteur contient *ori 60* comme origine de réplication dérivée de *Bt* et est le plasmide pEG853 logé dans la souche EG7529 de *E. coli*, déposée à l'Agricultural Research Culture Collection (NRRL) et ayant reçu le numéro d'inscription NRRL B-18631.

18. Vecteur navette de plasmide selon la revendication 16, caractérisé en outre en ce que le vecteur contient *ori 43* comme origine de réplication dérivée de *Bt* et est le plasmide pEG854 logé dans la souche EG7534 de *E. coli,* déposée à l'Agricultural Research Culture Collection (NRRL) et ayant reçu le numéro d'inscription NRRL B-18632.

19. Vecteur navette de plasmide selon l'une quelconque des revendications 1 à 15, caractérisé en outre en ce que le site de clonage multiple a des sites de coupure par les endonucléases de restriction dans l'ordre suivant, *BamHI XhoI PstI SstI SphI SmaI HindIII EcoRI SalI NotI*, et le site *BamHI* est adjacent à l'origine de réplication dérivée de *Bt*.

20. Vecteur navette de plasmide selon la revendication 19, caractérisé en outre en ce que le vecteur contient *ori 44* comme origine de réplication dérivée de *Bt* et est le plasmide pEG597 logé dans la souche EG1597 de *E. coli*, déposée à l'Agricultural Research Culture Collection (NRRL) et ayant reçu le numéro d'inscription NRRL B-18630.

21. Souche de *Bt* recombinante, caractérisée en ce qu'elle a un plasmide recombinant codant pour une toxine, fonctionnel, dérivant du vecteur navette de plasmide selon l'une quelconque des revendications 1 à 20.

22. Souche de *Bt* recombinante selon la revendication 21, caractérisée en outre en ce que la souche de *Bt* recombinante et son plasmide recombinant fonctionnel sont pratiquement exempts d'ADN de source biologique autre que *Bt*.

23. Souche de *Bt* recombinante selon les revendications 21 ou 22, caractérisée en outre en ce que le plasmide recombinant fonctionnel se compose essentiellement d'une origine de réplication dérivée de *Bt* et d'au moins un gène de toxine de *Bt* inséré dans un site de clonage multiple.

24. Fragment d'ADN isolé, purifié, caractérisé en ce qu'il est désigné sous le nom d'*ori* 44, en ce qu'il est utilisable comme origine de réplication fonctionnelle dans *Bt*, codant pour la séquence d'amino acide représentée dans la figure 5.

25. Fragment d'ADN isolé, purifié selon la revendication 24, caractérisé en outre en ce que la région de codage s'étend des bases de nucléotides 797 à 1732 dans la séquence de base de nucléotides représentée dans la figure 5.

26. Fragment d'ADN isolé, purifié, caractérisé en ce qu'il est désigné par *ori 60*, en ce qu'il est utilisable comme origine de réplication fonctionnelle dans *Bt*, codant pour la séquence d'amino acides représentée dans la figure 6.

27. Fragment d'ADN isolé, purifié selon la revendication 26, dans lequel la région de codage s'étend des bases de nucléotides 627 à 1844 dans la séquence de base des nucléotides représentée dans la figure 6.

28. Fragment d'ADN isolé, purifié, caractérisé en ce qu'il est désigné par *ori 43*, en ce qu'il est utilisable comme origine de réplication fonctionnelle dans *Bt*, codant pour la séquence d'amino acides représentée dans la figure 7.

29. Fragment d'ADN isolé, purifié selon la revendication 28, caractérisé en outre en ce que la région de codage s'étend des bases de nucléotides 829 à 2358 dans la séquence de base des nucléotides représentée dans la figure 7.

30. Plasmide recombinant caractérisé en ce qu'il contient le fragment d'ADN selon l'une quelconque des revendications 24 à 29.

31. Culture biologiquement pure d'une bactérie *Bacillus thuringiensis*, caractérisée en ce qu'elle est transformée par le plasmide recombinant selon la revendication 30.

32. Procédé de préparation d'un fragment d'ADN selon l'une quelconque des revendications 24 à 29 qui comprend le fait d'isoler ce fragment d'un vecteur le contenant en utilisant des enzymes de restriction appropriées et de le purifier par des procédés appropriés.
